(19) Europäisches Patentamt
European Patent Office
Office européen des brevets

(11) EP 1 406 859 B1

(12) **EUROPÄISCHE PATENTSCHRIFT**

(45) Veröffentlichungstag und Bekanntmachung des Hinweises auf die Patenterteilung:
**02.01.2008 Patentblatt 2008/01**

(21) Anmeldenummer: **02764691.8**

(22) Anmeldetag: **15.07.2002**

(51) Int Cl.:
***C07C 215/44*** $^{(2006.01)}$ ***C07D 295/096*** $^{(2006.01)}$
***C07D 209/14*** $^{(2006.01)}$ ***C07D 333/58*** $^{(2006.01)}$
***C07D 307/81*** $^{(2006.01)}$ ***A61K 31/133*** $^{(2006.01)}$
***A61P 25/00*** $^{(2006.01)}$

(86) Internationale Anmeldenummer:
**PCT/EP2002/007849**

(87) Internationale Veröffentlichungsnummer:
**WO 2003/008371 (30.01.2003 Gazette 2003/05)**

(54) **SUBSTITUIERTE 4-AMINOCYCLOHEXANOLDERIVATE**

SUBSTITUTED 4-AMINOCYCLOHEXANOL DERIVATIVES

DERIVES SUBSTITUES DE 4-AMINOCYCLOHEXANOL

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **17.07.2001 DE 10135637**
**17.07.2001 DE 10135635**

(43) Veröffentlichungstag der Anmeldung:
**14.04.2004 Patentblatt 2004/16**

(73) Patentinhaber: **Grünenthal GmbH**
**52078 Aachen (DE)**

(72) Erfinder:
- **SUNDERMANN, Bernd**
  **52066 Aachen (DE)**
- **HENNIES, Hagen-Heinrich**
  **52152 Simmerath (DE)**
- **KÖGEL, Babette-Yvonne**
  **52379 Langerwehe (DE)**
- **WNENDT, Stephan**
  **52066 Aachen (DE)**

(56) Entgegenhaltungen:
**WO-A-01/12195** **DE-A- 2 839 891**
**DE-A- 19 963 175** **US-A- 4 346 101**

- **F. VONVOIGTLANDER ET AL: "4-(p-Bromophenyl)-4-(dimethylamino)-1-phenethylcyclohexanol, an Extremely Potent Representative of a New Analgesic Series" JOURNAL OF MEDICINAL CHEMISTRY, Bd. 22, Nr. 10, 1979, Seiten 1157-1158, XP002216903 in der Anmeldung erwähnt**
- **KAWAMOTO H ET AL: "Synthesis of J-113397, the first potent and selective ORL1 antagonist" TETRAHEDRON, ELSEVIER SCIENCE PUBLISHERS, AMSTERDAM, NL, Bd. 57, Nr. 6, 4. Februar 2001 (2001-02-04), Seiten 981-986, XP004316527 ISSN: 0040-4020**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 406 859 B1

## Beschreibung

Die vorliegende Erfindung betrifft substituierte 4-Aminocyclohexanolderivate, Verfahren zu deren Herstellung, Arzneimittel enthaltend diese Verbindungen und die Verwendung von substituierten 4-Aminocyclohexanolderivaten zur Herstellung von Arzneimitteln zur Behandlung diverser Indikationen, insbesondere von Schmerz.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem $K_d$-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535). Auch auf der zellulären Ebene sind funktionelle Ähnlichkeiten der $\mu$, $\kappa$ und $\delta$ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin hemmt die Aktivität Kainat- oder Glutamat-stimulierter Hinterwurzelganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin nachgewiesen werden (Yamamoto und Nozaki-Taguchi, Anesthesiology, 87, 1997), die insofern besonders interessant ist, als das die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles, November 7.-12, 1998) und Diurese (Kapista et al., Life Sciences, 60, 1997, PL 15-21). In einem Übersichtsartikel von Calo et al. (Br.J. Pharmacol., 129, 2000, 1261 -1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Morphinen, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, aterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika aber auch als Antitussiva diskutiert.

Entsprechend vielfältig sind die Anwendungsmöglichkeiten von Verbindungen, die an den ORL1-Rezeptor binden und diesen aktivieren oder inhibieren.

Aufgabe der vorliegenden Erfindung war es, Wirkstoffe zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Ein Gegenstand der Erfindung sind daher substituierte 4-Aminocyclohexanolderivate gemäß der allgemeinen Formel I,

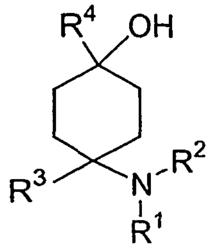

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{3-8}$-Cycloalkyl, unsubstituiert oder einfach oder mehrfach substituiert; oder über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$ mit Y = O, S oder $H_2$,

mit $R^6$ ausgewählt aus

H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;

und mit R7 ausgewählt aus

H; $C_{3-8}$-Cycloalkyl, Aryl oder Heteroaryl, jeweils unsubstituiert oder einfach oder mehrfach substituiert,

mit $R^8$ ausgewählt aus

Aryl oder Heteroaryl, jeweils unsubstituiert oder einfach oder mehrfach substituiert,

mit L ausgewählt aus

$$-C(O)-NH-, -NH-C(O)-, -C(O)-O-, -O-C(O)-, -O-, -S- \text{ oder } -S(O)_2-$$

mit $R^9$ ausgewählt aus

Aryl oder Heteroaryl, jeweils unsubstituiert oder einfach oder mehrfach substituiert,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Alle diese erfindungsgemäßen Verbindungen bzw. Verbindungsgruppen zeigen hervorragende Bindung an den ORL1-Rezeptor.

Verbindungen, die eine gewisse entfernte strukturelle Verwandtschaft mit den hier vorgeschlagenen Verbindungen zeigen, sind aus folgenden Schriften bekannt:

- Der DE-OS-28 39 891 bzw. dem parallelen US-Patent US 4,366,172 (Lednicer et al.). Darin werden die genannten

Verbindungen als analgetisch wirksam beschrieben, ohne daß Bezug auf den ORL1-Rezeptor genommen wird.

■ Den parallelen Artikeln:

- D. Lednicer und P.F. von Voightlander, J. Med. Chem. 1979, 22, 1157,
- D. Lednicer, P.F. von Voightlander und D.E. Emmert, J. Med. Chem. 1980, 23, 424, und
- D. Lednicer, P.F. von Voightlander und D.E. Emmert, J. Med. Chem. 1981, 24, 404,
- D. Lednicer, P.F. von Voightlander und D.E. Emmert, J. Med. Chem. 1981, 24, 340,
- P.F. VonVoightlander, D. Lednicer, R.A. Lewis und D.D. Gay, "Endogenous and Exogenous Opiate Agonists and Antagonists", Proc. Int. Narc. Res. Club Conf. (1980), Meeting Date 1979, Way E.Long (Ed), Publisher: Pergamon, Elmsford, N.Y.International, Pergamon, 1980, 17-21,

■ Kamenka et al., EurJ.Med.Chem.Chim.Ther.; FR; 19;3;1984;255-260 und

■ Rao M.N.A. und Rao S.C. Indian Drugs, 1985, 22 (5), 252-257.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$,$-CH_2-CH_2-$, $-CH_2-CH_2-CH_2-$ und $-CH_2-CH_2-CH_2-CH_2-$ zu verstehen, unter $(CH_2)_{4-5}$ ist-$CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{82}$, $OR^{82}$ einem Halogen, vorzugsweise F und/oder Cl, einem $CF_3$, einem CN, einem $NO_2$, einem $NR^{83}R^{84}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem

$C_{2-6}$-Alkylen.

Dabei steht der Rest $R^{82}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{83}$ und $R^{84}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{83}$ und $R^{84}$ bedeuten zusammen $CH_2CH_2OCH_2CH_2$, $CH_2CH_2NR^{85}CH_2CH_2$ oder $(CH_2)_{3-6}$, und der Rest $R^{85}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter physiologisch verträglich ist zu verstehen, daß die Substanz, insbesondere das Salz als solches, bei Anwendung im Menschen oder Säugetier verträglich ist, also beispielsweise nicht unphysiologisch (z.B. giftig) wirkt.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesaure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - verträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

In Bezug auf die oben beschriebenen erfindungsgemäßen substituierten 4-Aminocyclohexanolderivate ist es bevorzugt, wenn

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen, oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

In Bezug auf die oben beschriebenen erfindungsgemäßen substituierten 4-Aminocyclohexanolderivate ist es bevorzugt, wenn
$R^3$ ausgewählt ist aus $C_{5-6}$-Cycloalkyl, unsubstituiert oder einfach oder mehrfach substituiert; oder über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus über eine gesättigte, unverzweigte $C_{1-2}$-AlkylGruppe gebundenem Phenyl, Pyridyl, Furyl oder Thiophenyl, jeweils unsubstituiert oder einfach oder mehrfach substituiert.

In Bezug auf die oben beschriebenen erfindungsgemäßen substituierten 4-Aminocyclohexanolderivate ist es bevorzugt, wenn
$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$
vorzugsweise
$R^4$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; oder $-R^8-L-R^9$
insbesondere
$R^4$ ausgewählt ist aus Cyclopentyl, Cyclohexyl, Cycloheptyl, Cyclooctyl, Anthracenyl, Indolyl, Naphthyl, Benzothiazolyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert; oder $-R^8-L-R^9$.

In Bezug auf die direkt voranstehende bevorzugte Ausführungsform bezüglich $R^4$ ist es weiter bevorzugt, wenn
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus

$$-C(O)-NH-, -NH-C(O)-, -C(O)-O-, -O-C(O)-, -O-, -S- \text{ oder } -S(O)_2-,$$

und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise
$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus

$$-C(O)-NH-, -NH-C(O)-, -C(O)-O-, -O-C(O)- \text{ oder } -S(O)_2-,$$

und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus

$-S(O)_2-$

und $R^9$ ausgewählt ist aus
Phenyl unsubstituiert.
In einer witeren Ausführungsform ist es bevorzugt, wenn bezüglich der beschriebenen erfindungsgemäßen substituierten 4-Aminocyclohexanolderivate
$R^4$ ausgewählt ist aus $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$
mit Y = O, S oder $H_2$,
vorzugsweise
$R^4$ ausgewählt ist aus $-CHR^6R^7$, $-CHR^6-CH_2R^7$, $-CHR^6-CH_2-CH_2R^7$, $-C(Y)R^7$, $-C(Y)-CH_2R^7$ oder $-C(Y)-CH_2-CH_2R^7$
mit Y = O oder S,
insbesondere
$R^4$ ausgewählt ist aus $-CHR^6R^7$, $-CHR^6-CH_2R^7$, $-C(Y)R^7$ oder $-C(Y)-CH_2R^7$
mit Y=O.

In Bezug auf die direkt voranstehende bevorzugte Ausführungsform bezüglich $R^4$ ist es weiter bevorzugt, wenn $R^6$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$.

In Bezug auf die direkt voranstehende bevorzugte Ausführungsform bezüglich $R^4$ ist es weiter ebenfalls bevorzugt, wenn
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

In Bezug auf die beschriebenen erfindungsgemäßen substituierten 4-Aminocyclohexanolderivate ist es bevorzugt, wenn sie ausgewählt sind aus der folgenden Gruppe:

- 4-Benzyl-4-dimethylamino-1-phenethylcyclohexanol sowie dem entsprechenden Hydrochlorid,
- 4-Dimethylamino-1,4-diphenethylcyclohexanol sowie dem entsprechenden Hydrochlorid,
- 4-Benzyl-4-dimethylamino-1-[2-(2-fluorphenyl)ethyl]cyclohexanol sowie dem entsprechenden Hydrochlorid,
- 4-Benzyl-4-dimethylamino-1-[2-(4-fluorphenyl)ethyl]cyclohexanol sowie dem entsprechenden Hydrochlorid,
- 4-Dimethylamino-4-(2-fluorbenzyl)-1-phenethylcyclohexanol sowie dem entsprechenden Hydrochlorid,
- 4-Dimethylamino-4-(3-fluorbenzyl)-1-phenethylcyclohexanol sowie dem entsprechenden Hydrochlorid,
- 4-Dimethylamino-4-(4-fluorbenzyl)-1-phenethylcyclohexanol sowie dem entsprechenden Hydrochlorid,

- 4-Benzyl-4-dimethylamino-1-[2-(3-fluorphenyl)ethyl]cyclohexanol sowie dem entsprechenden Hydrochlorid,
- 4-Benzyl-4-dimethylamino-1-(2-fluorbenzyl)cyclohexanol sowie dem entsprechenden Hydrochlorid,
- 4-(Allylmethylamino)-4-benzyl-1-phenethylcyclohexanol sowie dem entsprechenden Hydrochlorid,
- 4-Benzyl-4-dimethylamino-1-(3-fluorbenzyl)cyclohexanol sowie dem entsprechenden Hydrochlorid,
- 4-Benzyl-4-dimethylamino-1-(4-fluorbenzyl)cyclohexanol sowie dem entsprechenden Hydrochlorid,
- 1-Benzyl-4-dimethylamino-4-(3-fluorbenzyl)cyclohexanol sowie dem entsprechenden Hydrochlorid oder
- 4-Benzyl-1-phenethyl-4-pyrrolidin-1-ylcyclohexanol sowie dem entsprechenden Hydrochlorid
- 4-Benzyl-4-dimethylamino-1-(1-methyl-1H-indol-2-yl)cyclohexanol
- 1-Benzo[b]thiophen-2-yl-4-benzyl-4-dimethylaminocyclohexanol
- 1-Benzo[b]thiophen-3-yl-4-benzyl-4-dimethylaminocyclohexanol
- 1-Benzofuran-2-yl-4-benzyl-4-dimethylamino-cyclohexanol

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Substanzen sind toxikologisch unbedenklich, so daß sie sich als pharmazeutischer Wirkstoff in Arzneimittel eignen.

Ein weiterer Gegenstand der Erfindung sind daher Arzneimittel enthaltend wenigstens ein erfindungsgemäßes substituiertes 4-Aminocyclohexanol-derivat gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten 4-Aminocyclohexanolderivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 4-Aminocyclohexanolderivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 4-Aminocyclohexanolderivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten 4-Aminocyclohexanolderivats appliziert.

Für alle vorstehenden Formen der erfindungsgemäßen Arzneimittel ist es besonders bevorzugt, wenn das Arzneimittel neben wenigstens einem substituierten 4-Aminocyclohexanolderivat noch ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes 4-Aminocyclohexanolderivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße substituierte 4-Aminocyclohexanolderivate zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist daher die Verwendung eines erfindungsgemäßen substituierten 4-Aminocyclohexanolderivats gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;in dargestellter Form oder in Form seiner Säuren oder

seiner Basen oder in Form seiner Salze, insbesondere der physiologisch verträglichen Salze, oder in Form seiner Solvate, insbesondere der Hydrate, zur Herstellung eines Arzneimittels zur Behandlung von Schmerz, insbesondere von akutem, viszeralem, neuropathischem oder chronischem Schmerz.

Wie bereits in der Einleitung ausgeführt, spielt der ORL1-Rezeptor neben der Funktion im Schmerzgeschehen noch in einer Vielzahl anderer physiologischer Prozeße insbesondere von medizinisch relevanter Bedeutung eine Rolle.

Daher ist ein weiterer Gegenstand der Erfindung die Verwendung eines erfindungsgemäßen substituierten 4-Aminocyclohexanolderivats gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;in dargestellter Form oder in Form seiner Säuren oder seiner Basen oder in Form seiner Salze, insbesondere der physiologisch verträglichen Salze, oder in Form seiner Solvate, insbesondere der Hydrate, zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum, Antitussivum oder Anesthetikum bzw. zur Coadministration bei Behandlung mit einem opioiden Analgetikum oder mit einem Anesthetikum, zur Diurese oder Antinatriurese und/oder Anxiolyse.

Dabei kann es in einer der vorstehenden Verwendungen bevorzugt sein, wenn ein verwendetes substituiertes 4-Aminocyclohexanolderivat als reines Diastereomer und/oder Enantiomer, als Razemat oder als nicht-äquimolare oder äquimolare Mischung der Diastereomere und/oder Enantiomere vorliegt und/oder neben dem substituierten 4-Aminocyclohexanolderivat noch ein Opioid, vorzugsweise ein starkes Opioid, insbesondere Morphin, oder ein Anesthetikum, vorzugsweise Hexobarbital oder Halothan, verwendet wird.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexan-1,4-diaminderivats oder eines erfindungsgemäßen Arzneimittels.

Ein weiterer Gegenstand der Erfindung ist ein Verfahren zur Herstellung der erfindungsgemäßen substituierten 4-Aminocyclohexanolderivate wie in der folgenden Beschreibung und Beispielen ausgeführt.

Insbesondere geeignet ist dabei ein Verfahren mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

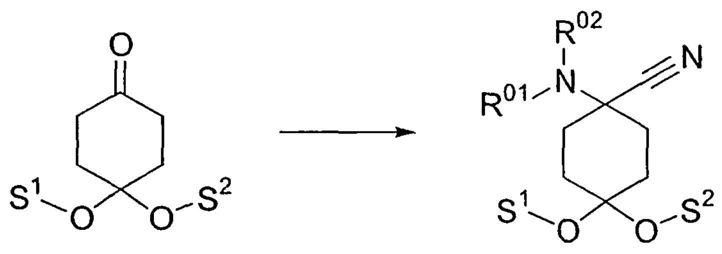

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

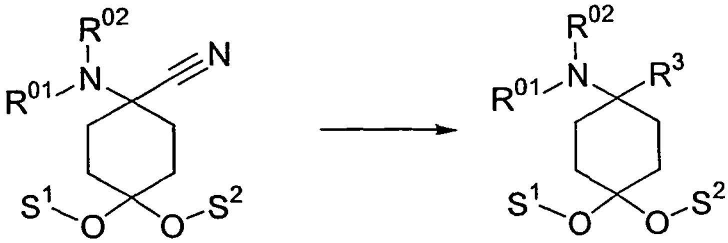

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa gemäß Formel III werden die Schutzgruppen $S^1$ und $S^2$ abgespalten, so daß ein 4-substituiertes 4-Aminocyclohexanonderivat gemäß Formel IV entsteht;

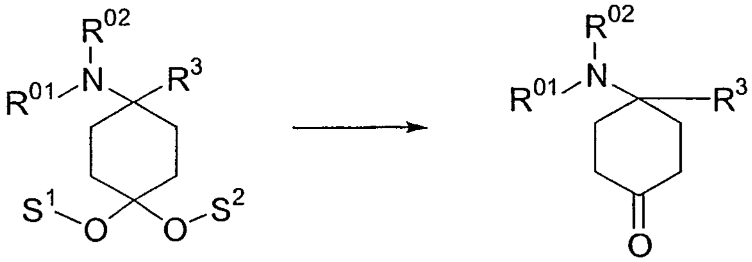

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IV mit metallorganischen Reagenzien, bevorzugt Grignard- oder Organolithiumreagenzien, der Formel Metall-$R^3$ umgesetzt, so daß eine Verbindung gemäß Formel V entsteht;

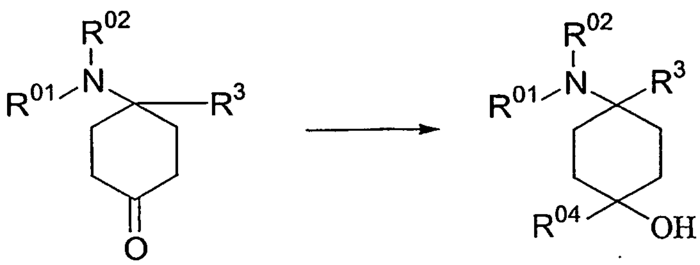

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,
wobei $R^1$, $R^2$, $R^3$ $R^4$ und $R^5$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{02}$ zusammen einen Ring bilden und $CH_2CH_2OCH_2CH_2$, $CH_2CH_2NR^{05}CH_2CH_2$ oder $(CH_2)_{3-6}$ bedeuten,
mit $R^{05}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$
mit Y = O, S oder $H_2$,
mit $R^6$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit R7 ausgewählt aus
H; $C_{3-8}$-Cycloalkyl, Aryl oder Heteroaryl, jeweils unsubstituiert oder einfach oder mehrfach substituiert,
mit $R^8$ ausgewählt aus
Aryl oder Heteroaryl, jeweils unsubstituiert oder einfach oder mehrfach substituiert,
mit L ausgewählt aus

$$-C(O)-NH-, -NH-C(O)-, -C(O)-O-, -O-C(O)-, -O-, -S- \text{ oder } -S(O)_2-$$

mit $R^9$ ausgewählt aus
Aryl oder Heteroaryl, jeweils unsubstituiert oder einfach oder mehrfach substituiert,
und $S^1$ und $S^2$ unabhängig voneinander ausgewählt sind aus Schutzgruppen oder zusammen eine Schutzgruppe bedeuten, vorzugsweise Monoacetal.

In Bezug auf das beschriebene besonders geeignete Verfahren ist es besonders bevorzugt, wenn die Schutzgruppen am H bei $R^{01}$, $R^{02}$, $R^{04}$ und/oder $R^{05}$ ausgewählt sind aus Alkyl, Benzyl oder Carbamaten, beispielsweise FMOC, Z oder Boc.
Im folgenden wird die Erfindung weiter durch Beispiele erläutert, ohne sie darauf zu beschränken.

## Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.
Die Analytik erfolgte über NMR-Spektroskopie, gegebenenfalls in Kombination mit anderen analytischen Verfahren wie Dünnschichtchromatographie, Massenspektrometrie oder HPLC.

**Beispiel 1**

Allgemeine Möglichkeit der Herstellung erfindungsgemäßer Verbindungen

Die Herstellung dieser Verbindungen erfolgt ausgehend von einem geeignet als beispielsweise Monoacetal geschützten Cyclohexan-1,4-dion II. Durch Umsetzung mit Kaliumcyanid in Gegenwart eines sekundären Amins wird ein geschütztes N-substituiertes 1-Amino-4-oxo-cyclohexancarbonitrilderivat III erhalten.

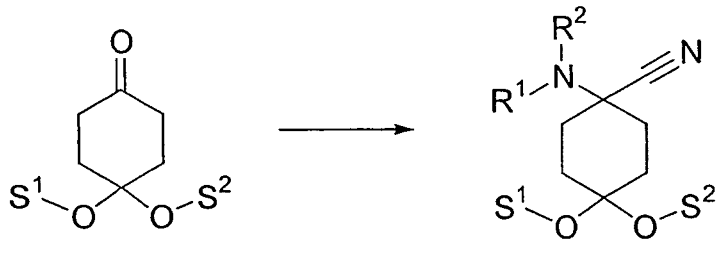

II III

Die Umsetzung des Aminonitrils III mit metallorganischen Reagenzien, bevorzugt Grignard- oder Organolithiumreagenzien, bewirkt eine Substitution der Nitrilfunktion, so daß nach anschließender Abspaltung der Carbonylschutzgruppe ein 4-substituiertes 4-Aminocyclohexanonderivat IV erhalten wird.

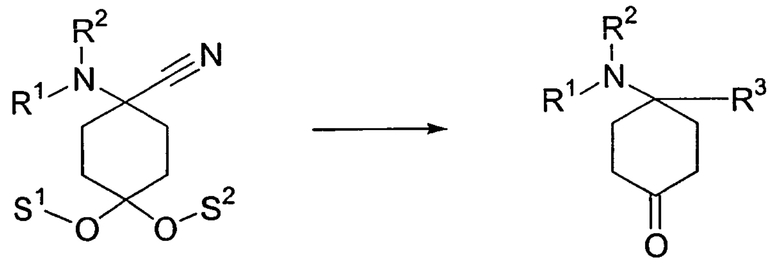

III IV

Intermediate des Typs IV können schließlich durch Addition metallorganischer Reagenzien, bevorzugt Grignard- oder Organolithiumreagenzien, in erfindungsgemäße 4-Aminocyclohexanolderivate I überführt werden.

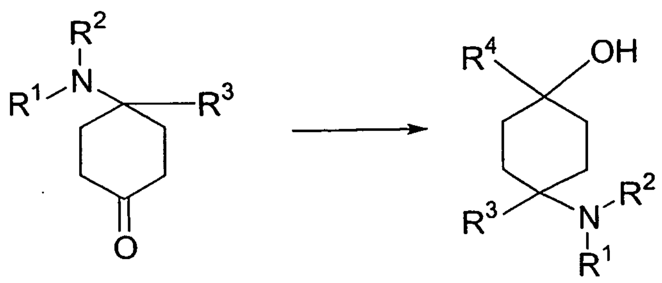

IV I

**Beispiel 2**

Messung der ORL1-Bindunq

Die 4-Aminocyclohexanolderivate der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als $K_i$-Wert angegeben.

| **Beispiel** | | **$K_i$-Wert** |
|---|---|---|
| Nr. | | (in μmol) |
| 4 | | 0,02 |
| 5 | | |
| 6 | | 0,03 |
| 7 | | 0,04 |
| 8 | | 0,05 |
| 9 | | 0,03 |
| 10 | | 0,20 |
| 11 | | 0,02 |
| 12 | | |
| 13 | | 0,06 |
| 14 | | 0,90 |
| 15 | | 0,40 |
| 16 | | 0,89 |
| 17 | | 0,04 |
| 18 | | 0,13 |
| 19 | | 0,045 |
| 20 | | 0,15 |
| 21 | | 0,15 |

**Beispiel 3**

Analgesieprüfung im Tail-Flick Test an der Maus

Die analgetische Wirksamkeit der erfindungsgemäßen Verbindungen wurde im Brennstrahl (Tail-flick) Test an der Maus nach der Methode von D'Amour and Smith (J. Pharm. Exp. Ther. 72, 74 79 (1941) untersucht. Dazu wurden NMRI-Mäuse mit einem Gewicht zwischen 20 - 24 g verwendet. Die Tiere wurden einzeln in spezielle Testkäfige gesetzt und die Schwanzbasis einem focussierten Wärmestrahl einer elektrischen Lampe (Tail-flick Typ 55/12/10.fl, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 - 5 Sekunden betrug. Vor Gabe einer erfindungsgemäßen Verbindung wurden die Tiere innerhalb von fünf Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).

Zur Bestimmung der Dosisabhängigkeit wurde die jeweilige erfindungsgemäße Verbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum 20 Minuten nach intravenöser Substanzgabe.

Die untersuchten erfindungsgemäßen Verbindungen zeigten eine ausgeprägte analgetische Wirkung. Die Ergebnisse sind in der nachfolgenden Tabelle zusammengefaßt.

| **Beispiel** | | **% MPE** | **$ED_{50}$** |
|---|---|---|---|
| Nr. | | (Dosierung in mg/kg intravenös) | mg/kg intravenös |
| 4 | | 100 (1) | 0,015 |
| 5 | | 100 (1) | 0,040 |
| 6 | | 98 (1) | 0,055 |
| 7 | | 93 (1) | 0,10 |
| 8 | | 97 (1) | 0,093 |
| 9 | | 100 (1) | 0,089 |
| 10 | | 97 (1) | 0,16 |
| 11 | | 99 (1) | 0,059 |
| 12 | | 90 (1) | |
| 13 | | 97 (10) | |
| 14 | | 78 (1) | |
| 15 | | 92 (1) | |
| 16 | | 100 (1) | 0,028 |
| 17 | | 100 (1) | 0,15 |
| 18 | | 63 (10) | |
| 19 | | 100(1) | 0,04 |

**Beispiel 4**

4-Benzyl-4-dimethylamino-1-phenethylcyclohexanol Hydrochlorid

200 g 1,4-Dioxaspiro[4.5]decan-8-on wurden vorgelegt, nacheinander 1,68 l wässrige Dimethylaminlösung (40 Volumenprozent), 200 ml Methanol, 200 g Kaliumcyanid und 303 g Dimethylamin Hydrochlorid zugegeben und die Reaktionsmischung für 65 Stunden bei Raumtemperatur gerührt. Die erhaltene weiße Suspension wurde viermal mit je 800 ml Diethylether extrahiert, die vereinigten Extrakte zunächst eingeengt und mit 500 ml Dichlormethan aufgenommen, die organische Phase abgetrennt, über Natriumsulfat getrocknet, filtriert, eingeengt und im Vakuum weitgehend von Lösungsmittelresten befreit. Es wurden 265 g 8-Dimethylamino-1,4-dioxaspiro[4.5]decan-8-carbonitril als weißer Feststoff erhalten.

50 g 8-Dimethylamino-1,4-dioxaspiro[4.5]decan-8-carbonitril wurden in 400 ml Tetrahydrofuran p.a. gelöst, unter Stickstoffatmosphäre 214 ml 2,0 molare Benzylmagnesiumchloridlösung in THF zugegeben und über Nacht bei Raumtemperatur gerührt. Zur Aufarbeitung wurden unter Eiskühlung 200 ml gesättigte Ammoniumchloridlösung zugegeben, die Phasen getrennt, die wässrige Phase zweimal mit je 250 ml Diethylether extrahiert, die vereinigten organischen Phasen über Natriumsulfat getrocknet, filtriert, eingeengt und im Vakuum weitgehend von Lösungsmittelresten befreit.

Das erhaltene rohe (8-Benzyl-1,4-dioxaspiro[4.5]dec-8-yl)-dimethylamin (78,4 g) wurde ohne weitere Aufreinigung für 24 Stunden mit einem Gemisch aus 200 ml konz. Salzsäure (32 Massenprozent) und 120 ml Wasser bei Raumtemperatur gerührt. Anschließend wurde die Reaktionsmischung zunächst dreimal mit je 100 ml Diethylether gewaschen, dann unter Eiskühlung durch Zugabe von Natronlauge (32 Massenprozent) alkalisiert, dreimal mit je 100 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 50,4 g 4-Benzyl-4-dimethylaminocyclohexanon als bräunlicher Feststoff erhalten.

25,0 g 4-Benzyl-4-dimethylaminocyclohexanon wurden in 150 ml Tetrahydrofuran p.a. gelöst, bei Eisbadkühlung unter Stickstoffatmosphäre 151 ml 1,0 molare Phenethylmagnesiumchloridlösung in THF zugegeben und über Nacht bei Raumtemperatur gerührt. Zur Aufarbeitung wurden unter Eiskühlung 150 ml Ammoniumchloridlösung (20 Massenprozent) zugegeben, die Phasen getrennt, die wässrige Phase dreimal mit je 80 ml Diethylether extrahiert, die vereinigten organischen Phasen dreimal mit je 70 ml verdünnter Salzsäure (5 Massenprozent) extrahiert, die vereinigten wässrigen Extrakte mit 50 ml Diethylether gewaschen, mit Ammoniaklösung (25 Massenprozent) auf ph 9 eingestellt, dreimal mit je 80 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (32,6 g) wurde mit Diethylether/Hexan (V/V = 1:1) an Kieselgel chromatographiert. 3,5 g des erhaltenen unpolareren Diastereoisomers von 4-Benzyl-4-dimethylamino-1-phenethylcyclohexanol wurden in 28 ml 2-Butanon gelöst, bei Raumtemperatur 103 µl Wasser gefolgt von 1,44 ml Trimethylchlorsilan zugegeben und über Nacht bei Raumtemperatur gerührt. Der ausgefallene weiße Feststoff wurde abgesaugt, mit Diethylether gewaschen und im Ölpumpenvakuum getrocknet. Es wurden 2,47 g des Hydrochlorids des unpolareren Diastereoisomers von 4-Benzyl-4-dimethylamino-1-phenethylcyclohexanol erhalten.

**Beispiel 5**

4-Dimethylamino-1,4-diphenethylcyclohexanol Hydrochlorid

45 g 8-Dimethylamino-1,4-dioxaspiro[4.5]decan-8-carbonitril wurden in 250 ml Tetrahydrofuran p.a. gelöst, unter Stickstoffatmosphäre 238 ml 1,0 molare Phenethylmagnesiumchloridlösung in THF zugegeben und über Nacht bei Raumtemperatur gerührt. Zur Aufarbeitung wurden unter Eiskühlung 100 ml Ammoniumchloridlösung (20 Massenprozent) zugegeben, die Phasen getrennt, die wässrige Phase zweimal mit je 200 ml Diethylether extrahiert, die vereinigten organischen Phasen nacheinander mit 100 ml Wasser und 100 gesättigter Natriumchloridlösung gewaschen, über Natriumsulfat getrocknet, filtriert, eingeengt und im Vakuum weitgehend von Lösungsmittelresten befreit. Das als gelbes Öl erhaltene rohe Dimethyl-(8-phenethyl-1,4-dioxa-spiro[4.5]dec-8-yl)-amin (54,1 g) wurde ohne weitere Aufreinigung für 24 Stunden mit einem Gemisch aus 120 ml konz. Salzsäure (32 Massenprozent) und 70 ml Wasser bei Raumtemperatur gerührt. Anschließend wurde die Reaktionsmischung zunächst dreimal mit je 50 ml Diethylether gewaschen, dann unter Eiskühlung durch Zugabe von Natronlauge (32 Massenprozent) alkalisiert, dreimal mit je 100 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 35,7 g rohes 4-Dimethylamino-4-phenethyl-cyclohexanon als langsam kristallisierendes, braunes Öl erhalten.

7,58 g 4-Dimethylamino-4-phenethylcyclohexanon wurden in 45 ml Tetrahydrofuran p.a. gelöst, bei Eisbadkühlung unter Stickstoffatmosphäre 43 ml 1,0 molare Phenethylmagnesiumchloridlösung in THF zugegeben und über Nacht bei Raumtemperatur gerührt. Zur Aufarbeitung wurden unter Eiskühlung 43 ml Ammoniumchloridlösung (20 Massenprozent) zugegeben und dreimal mit je 80 ml Diethylether extrahiert, die vereinigten organischen Phasen dreimal mit je 70 ml verdünnter Salzsäure (5 Massenprozent) extrahiert, die vereinigten wässrigen Extrakte mit 50 ml Diethylether gewaschen, mit Ammoniaklösung (25 Massenprozent) auf ph 9 eingestellt, dreimal mit je 80 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (9,57 g) wurde mit Diethylether/Hexan (V/V = 1:1) an Kieselgel chromatographiert. 938 mg des erhaltenen unpolareren Diastereoisomers von 4-Dimethylamino-1,4-diphenethylcyclohexanol wurden in 7,5 ml 2-Butanon gelöst, bei Raumtemperatur 26 µl Wasser gefolgt von 371 µl Trimethylchlorsilan zugegeben und über Nacht bei Raumtemperatur gerührt. Der ausgefallene weiße Feststoff wurde abgesaugt, mit Diethylether gewaschen und im Ölpumpenvakuum getrocknet. Es wurden 1,00 g des Hydrochlorids des unpolareren Diastereoisomers von 4-Dimethylamino-1,4-diphenethylcyclohexanol erhalten.

**Beispiel 6**

4-Benzyl-4-dimethylamino-1-[2-(2-fluorphenyl)ethyl]cyclohexanol Hydrochlorid

11,4 g Lithiumaluminiumhydrid wurden in 100 ml Tetrahydrofuran p.a. vorgelegt, unter Stickstoffatmosphäre

zum Rückfluß erhitzt, 50 g 2-Fluorphenylessigsäure, gelöst in 400 ml Tetrahydrofuran p.a., zugetropft und die Reaktionsmischung weitere zwei Stunden erhitzt. Zur Aufarbeitung wurden bei Eisbadkühlung unter Rühren 72 ml Wasser gefolgt von 250 ml halbkonzentierter Salzsäure (16 Massenprozent) zugetropft. Es wurde zweimal mit je 250 ml Diethylether extrahiert, die vereinigten Extrakte zweimal mit je 100 ml Natriumhydrogencarbonatlösung (5 Massenprozent) gewaschen, über Natriumsulfat getrocknet, filtriert, eingeengt und im Vakuum weitgehend von Lösungsmittelresten befreit. Es wurden 40,2 g 2-(2-Fluorphenyl)ethanol erhalten.

In einem Edelstahlautoklaven wurden 50 g 2-(2-Fluorphenyl)ethanol, 19 ml konzentrierte Schwefelsäure und 58 ml wässrige Bromwasserstoffsäure (47 Massenprozent) über Nacht auf 100 °C erhitzt. Nach Abkühlung wurde mit 500 ml Wasser verdünnt, zweimal mit je 250 ml Dichlormethan extrahiert, die vereinigten Extrakte über Kaliumcarbonat getrocknet, filtriert, eingeengt und im Vakuum weitgehend von Lösungsmittelresten befreit. Es wurden 61,8 g 1-(2-Bromethyl)-2-fluorbenzol erhalten.

Unter Stickstoffatmosphäre wurden 624 mg Magnesium in 13 ml Tetrahydrofuran p.a. gerührt und ca. ein Drittel der Lösung von 4,69 g 1-(2-Bromethyl)-2-fluorbenzol in 13 ml Tetrahydrofuran p.a. zugegeben. Die restliche Lösung wurde nach dem Anspringen der Grignardbildung zügig zugetropft, nach beendeter Zugabe eine Stunde nachgerührt, anschließend 2,97 g 4-Benzyl-4-dimethylaminocyclohexanon, gelöst in 13 ml Tetrahydrofuran p.a., zugetropft und die Reaktionsmischung über Nacht gerührt. Zur Aufarbeitung wurden unter Eiskühlung 26 ml Ammoniumchloridlösung (20 Massenprozent) zugegeben, die Phasen getrennt, die wässrige Phase zweimal mit je 40 ml Diethylether extrahiert, die vereinigten organischen Phasen mit 50 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (4,39 g) wurde mit Diethylether/Hexan (V/V = 1:1) an Kieselgel chromatographiert. Es wurden 1,50 g des unpolareren Diastereoisomers von 4-Benzyl-4-dimethylamino-1-[2-(2-fluorphenyl)ethyl]cyclohexanol erhaltenen, aus dem wie für Beispiel 4 beschrieben 1,56 g des korrespondierenden Hydrochlorids hergestellt wurden.

## Beispiel 7

4-Benzyl-4-dimethylamino-1-[2-(4-fluorphenyl)ethyl]cyclohexanol Hydrochlorid

1-(2-Bromethyl)-4-fluorbenzol wurde, wie oben für 1-(2-Bromethyl)-2-fluorbenzol beschrieben, aus 4-Fluorphenylessigsäure hergestellt.

Unter Stickstoffatmosphäre wurden 841 mg Magnesium in 17 ml Tetrahydrofuran p.a. gerührt und ca. ein Drittel der Lösung von 6,32 g 1-(2-Bromethyl)-2-fluorbenzol in 17 ml Tetrahydrofuran p.a. zugegeben. Die restliche Lösung wurde nach dem Anspringen der Grignardbildung zügig zugetropft, nach beendeter Zugabe eine Stunde nachgerührt, anschließend 4,00 g 4-Benzyl-4-dimethylaminocyclohexanon, gelöst in 17 ml Tetrahydrofuran p.a., zugetropft und die Reaktionsmischung über Nacht gerührt. Zur Aufarbeitung wurden unter Eiskühlung 35 ml Ammoniumchloridlösung (20 Massenprozent) zugegeben, die Phasen getrennt, die wässrige Phase zweimal mit je 40 ml Diethylether extrahiert, die vereinigten organischen Phasen mit 50 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (4,39 g) wurde an Kieselgel chromatographiert. Es wurden 1,08 g des unpolareren Diastereoisomers von 4-Benzyl-4-dimethylamino-1-[2-(4-fluorphenyl)ethyl]cyclohexanol erhaltenen, aus dem wie für Beispiel 4 beschrieben 1,10 g des korrespondierenden Hydrochlorids hergestellt wurden.

## Beispiel 8

4-Dimethylamino-4-(2-fluorbenzyl)-1-phenethylcyclohexanol Hydrochlorid

Unter Stickstoffatmosphäre wurden 1,16 g Magnesium in 20 ml Diethylether p.a. gerührt und ca. ein Drittel der Lösung von 6,19 g 2-Fluorbenzylchlorid in 25 ml Diethylether p.a. zugegeben. Die restliche Lösung wurde nach dem Anspringen der Grignardbildung zügig zugetropft, nach beendeter Zugabe eine Stunde nachgerührt, anschließend eine Lösung von 5,00 g 8-Dimethylamino-1,4-dioxaspiro[4.5]decan-8-carbonitril in 25 ml Diethylether p.a. zugetropft und die Reaktionsmischung über Nacht gerührt. Zur Aufarbeitung wurden unter Eiskühlung 36 ml Ammoniumchloridlösung (20 Massenprozent) zugegeben, die Phasen getrennt, die wässrige Phase zweimal mit je 50 ml Diethylether extrahiert, die vereinigten organischen Phasen nacheinander mit 20 ml Wasser und 20 ml gesättigter Natriumchloridlösung gewaschen. Das erhaltene rohe [8-(2-Fluorbenzyl)-1,4-dioxaspiro[4.5]dec-8-yl]dimethylamin (7,34 g) wurde ohne weitere Aufreinigung für 24 Stunden mit einem Gemisch aus 18 ml konz. Salzsäure (32 Massenprozent) und 10 ml Wasser bei Raumtemperatur gerührt. Anschließend wurde die Reaktionsmischung zunächst dreimal mit je 50 ml Diethylether gewaschen, dann unter Eiskühlung durch Zugabe von wässrigem Ammoniak (25 Massenprozent) auf ph 9 eingestellt, dreimal mit je 50 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 5,80 g 4-Dimethylamino-4-(2-fluorbenzyl) cyclohexanon als gelber Feststoff erhalten. 5,79 g 4-Dimethylamino-4-(2-fluorbenzyl)cyclohexanon wurden in 35 ml Tetrahydrofuran p.a. gelöst, bei Eisbadkühlung unter Stickstoffatmosphäre 42 ml 1,0 molare Phenethylmagnesiumchloridlösung in THF zugegeben und über Nacht bei Raumtemperatur gerührt. Zur Aufarbeitung wurden unter Eiskühlung 42 ml Ammoniumchloridlösung (20 Massenprozent) zugegeben, die Phasen getrennt, die wässrige Phase dreimal mit je 50 ml Diethylether extrahiert, die vereinigten organischen Phasen mit 30 ml Wasser gewaschen, dreimal mit je 50 ml verdünnter Salzsäure (5 Massenprozent) extrahiert, die vereinigten wässrigen Extrakte mit 50 ml Diethylether gewaschen, mit Ammoniaklösung (25 Massenprozent) auf ph 9 eingestellt, dreimal mit je 50 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (7,76 g) wurde mit Diethylether/Hexan (V/V = 1:1) an Kieselgel chromatographiert. Es wurden 1,33 g des unpolareren Diastereoisomers von 4-Dimethylamino-4-(2-fluorbenzyl)-1-phenethylcyclohexanol erhaltenen, aus dem wie für Beispiel 4 beschrieben 536 mg des korrespondierenden Hydrochlorids hergestellt wurden.

**Beispiel 9**

4-Dimethylamino-4-(3-fluorbenzyl)-1-phenethylclohexanol Hydrochlorid

Unter Stickstoffatmosphäre wurden 925 mg Magnesium in 19 ml Diethylether p.a. gerührt und ca. ein Drittel der Lösung von 4,95 g 3-Fluorbenzylchlorid in 19 ml Diethylether p.a. zugegeben. Die restliche Lösung wurde nach dem Anspringen der Grignardbildung zügig zugetropft, nach beendeter Zugabe eine Stunde nachgerührt, anschließend eine Lösung von 4,00 g 8-Dimethylamino-1,4-dioxaspiro[4.5]decan-8-carbonitril in 25 mi Diethylether p.a. zugetropft und die Reaktionsmischung über Nacht gerührt. Zur Aufarbeitung wurden unter Eiskühlung 29 ml Ammoniumchloridlösung (20 Massenprozent) zugegeben, die Phasen getrennt, die wässrige Phase zweimal mit je 50 ml Diethylether extrahiert, die vereinigten organischen Phasen nacheinander mit 20 ml Wasser und 20 ml gesättigter Natriumchloridlösung gewaschen. Das erhaltene rohe [8-(3-Fluorbenzyl)-1,4-dioxa-spiro[4.5]dec-8-yl]dimethylamin (5,75 g gelber Feststoff) wurde ohne weitere Aufreinigung für 24 Stunden mit einem Gemisch aus 14 ml konz. Salzsäure (32 Massenprozent) und 8 ml Wasser bei Raumtemperatur gerührt. Anschließend wurde die Reaktionsmischung zunächst dreimal mit je 30 ml Diethylether gewaschen, dann unter Eiskühlung durch Zugabe von wässrigem Ammoniak (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 4,71 g 4-Dimethylamino-4-(3-fluorbenzyl)cyclohexanon als gelber Feststoff erhalten.

4,67 g 4-Dimethylamino-4-(3-fluorbenzyl)cyclohexanon wurden in 28 ml Tetrahydrofuran p.a. gelöst, bei Eisbadkühlung unter Stickstoffatmosphäre 34 ml 1,0 molare Phenethylmagnesiumchloridlösung in THF zugegeben und über Nacht bei Raumtemperatur gerührt. Zur Aufarbeitung wurden unter Eiskühlung 34 ml Ammoniumchloridlösung (20 Massenprozent) zugegeben, die Phasen getrennt, die wässrige Phase dreimal mit je 40 ml Diethylether extrahiert, die vereinigten organischen Phasen mit 25 ml Wasser gewaschen, dreimal mit je 40 ml verdünnter Salzsäure (5 Massenprozent) extrahiert, die vereinigten wässrigen Extrakte mit 25 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (6,12 g gelbes Harz) wurde mit Diethylether/Hexan (V/V = 1:1) an Kieselgel chromatographiert. Es wurden 1,28 g des unpolareren Diastereoisomers von 4-Dimethylamino-4-(3-fluorbenzyl)-1-phenethylcyclohexanol erhaltenen, aus dem wie für Beispiel 4 beschrieben 1,30 g des korrespondierenden Hydrochlorids hergestellt wurden.

**Beispiel 10**

4-Dimethylamino-4-(4-fluorbenzyl)-1-phenethylcyclohexanol Hydrochlorid

Unter Stickstoffatmosphäre wurden 925 mg Magnesium in 19 ml Diethylether p.a. gerührt und ca. ein Drittel der Lösung von 4,95 g 4-Fluorbenzylchlorid in 19 ml Diethylether p.a. zugegeben. Die restliche Lösung wurde nach dem Anspringen der Grignardbildung zügig zugetropft, nach beendeter Zugabe eine Stunde nachgerührt, anschließend eine Lösung von 4,00 g 8-Dimethylamino-1,4-dioxaspiro[4.5]decan-8-carbonitril in 25 ml Diethylether p.a. zugetropft und die Reaktionsmischung über Nacht gerührt. Zur Aufarbeitung wurden unter Eiskühlung 29 ml Ammoniumchloridlösung (20 Massenprozent) zugegeben, die Phasen getrennt, die wässrige Phase zweimal mit je 50 ml Diethylether extrahiert, die vereinigten organischen Phasen nacheinander mit 20 ml Wasser und 20 ml gesättigter Vatriumchloridlösung gewaschen.

Das erhaltene rohe [8-(4-Fluorbenzyl)-1,4-dioxa-spiro[4.5]dec-8-yl]dimethylamin (5,76 g gelber Feststoff) wurde ohne weitere Aufreinigung für 24 Stunden mit einem Gemisch aus 14 ml konz. Salzsäure (32 Massenprozent) und 8 ml Wasser bei Raumtemperatur gerührt. Anschließend wurde die Reaktionsmischung zunächst dreimal mit je 30 ml Diethylether gewaschen, dann unter Eiskühlung durch Zugabe von wässrigem Ammoniak (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 4,70 g 4-Dimethylamino-4-(4-fluorbenzyl)cyclohexanon als gelber Feststoff erhalten. 4,69 g 4-Dimethylamino-4-(4-fluorbenzyl)cyclohexanon wurden in 28 ml Tetrahydrofuran p.a. gelöst, bei Eisbadkühlung unter Stickstoffatmosphäre 34 ml 1,0 molare Phenethylmagnesiumchloridlösung in THF zugegeben und über Nacht bei Raumtemperatur gerührt. Zur Aufarbeitung wurden unter Eiskühlung 34 ml Ammoniumchloridlösung (20 Massenprozent) zugegeben, die Phasen getrennt, die wässrige Phase dreimal mit je 40 ml Diethylether extrahiert, die vereinigten organischen Phasen mit 25 ml Wasser gewaschen, dreimal mit je 40 ml verdünnter Salzsäure (5 Massenprozent) extrahiert, die vereinigten wässrigen Extrakte mit 25 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (6,40 g gelbes Harz) wurde mit Diethylether/Hexan (V/V = 1: 1) an Kieselgel chromatographiert. Es wurden 1 ,45 g des unpolareren Diastereoisomers von 4-Dimethylamino-4-(4-fluorbenzyl)-1-phenethylcyclohexanol erhaltenen, aus dem wie für Beispiel 4 beschrieben 1,44 g des korrespondierenden Hydrochlorids hergestellt wurden.

## Beispiel 11

4-Benzyl-4-dimethylamino-1-[2-(4-fluorphenyl)ethyl]cyclohexanol Hydrochlorid

1-(2-Bromethyl)-3-fluorbenzol wurde, wie oben für 1-(2-Bromethyl)-2-fluorbenzol beschrieben, aus 3-Fluorphenylessigsäure hergestellt.

Unter Stickstoffatmosphäre wurden 757 mg Magnesium in 15 ml Tetrahydrofuran p.a. gerührt und ca. ein Drittel der Lösung von 5,69 g 1-(2-Bromethyl)-3-fluorbenzol in 16 ml Tetrahydrofuran p.a. zugegeben. Die restliche Lösung wurde nach dem Anspringen der Grignardbildung zügig zugetropft, nach beendeter Zugabe eine Stunde nachgerührt, anschließend 3,60 g 4-Benzyl-4-dimethylaminocyclohexanon, gelöst in 16 ml Tetrahydrofuran p.a., zugetropft und die Reaktionsmischung über Nacht gerührt. Zur Aufarbeitung wurden unter Eiskühlung 31 ml Ammoniumchloridlösung (20 Massenprozent) zugegeben, die Phasen getrennt, die wässrige Phase zweimal mit je 40 ml Diethylether extrahiert, die vereinigten organischen Phasen mit 30 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (3,96 g) wurde mit Diethylether/Hexan (V/V = 1:1) an Kieselgel chromatographiert. Es wurden 301 mg des unpolareren Diastereoisomers von 4-Benzyl-4-dimethylamino-1-[2-(4-fluorphenyl)ethyl]cyclohexanol erhaltenen, aus dem wie für Beispiel 4 beschrieben 254 mg des korrespondierenden Hydrochlorids hergestellt wurden.

## Beispiel 12

4-Benzyl-4-dimethylamino-1-(2-fluorbenzyl)cyclohexanol Hydrochlorid

Unter Stickstoffatmosphäre wurden 757 mg Magnesium in 15 ml Diethylether p.a. gerührt und ca. ein Drittel der Lösung von 4,05 g 2-Fluorbenzylchlorid in 15 ml Diethylether p.a. zugegeben. Die restliche Lösung wurde nach dem Anspringen der Grignardbildung zügig zugetropft, nach beendeter Zugabe eine Stunde nachgerührt, anschließend 3,60 g 4-Benzyl-4-dimethylaminocyclohexanon, gelöst in 40 ml Diethylether p.a., zugetropft und die Reaktionsmischung über Nacht gerührt. Zur Aufarbeitung wurden unter Eiskühlung 31 ml Ammoniumchloridlösung (20 Massenprozent) zugegeben, die Phasen getrennt, die wässrige Phase zweimal mit je 40 ml Diethylether extrahiert, die vereinigten organischen Phasen mit 30 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (5,02 g) wurde mit Diethylether/Hexan (V/V = 1:1) an Kieselgel chromatographiert. Es wurden 2,44 g des unpolareren Diastereoisomers von 4-Benzyl-4-dimethylamino-1-(2-fluorbenzyl)cyclohexanol erhaltenen, aus dem wie für Beispiel 4 beschrieben 2,53 g des korrespondierenden Hydrochlorids hergestellt wurden.

**Beispiel 13**

4-(Allylmethylamino)-benzyl-1-phenethylcyclohexanol Hydrochlorid

Ein Gemisch aus 9 ml Wasser, 5,3 ml Salzsäure (32 Massenprozent), 8 ml Methanol, 17,5 g Allylmethylamin, 8,00 g 1,4-Dioxaspiro[4.5]decan-8-on und 8,0 g Kaliumcyanid wurde für 65 Stunden bei Raumtemperatur gerührt. Die erhaltene gelblich-weiße Suspension wurde viermal mit je 25 ml Diethylether extrahiert, die vereinigten Extrakte über Natriumsulfat getrocknet, filtriert, eingeengt und im Vakuum weitgehend von Lösungsmittelresten befreit. Es wurden 11,3 g 8-(Allylmethylamino)-1,4-dioxaspiro[4.5]decan-8-carbonitril als hellbraune Flüssigkeit erhalten.
Zu 14,8 ml 2,0 molarer Benzylmagnesiumchloridlösung in THF wurde unter Stickstoffatmosphäre eine Lösung von 3,50 g 8-(Allylmethylamino)-1,4-dioxa-spiro[4.5]decan-8-carbonitril in 35 ml Tetrahydrofuran p.a. zugetropft und über Nacht bei Raumtemperatur gerührt. Zur Aufarbeitung wurden unter Eiskühlung 25 ml gesättigte Ammoniumchloridlösung zugegeben, die Phasen getrennt, die wässrige Phase dreimal mit je 25 ml Diethylether extrahiert, die vereinigten organischen Phasen über Natriumsulfat getrocknet, filtriert, eingeengt und im Vakuum weitgehend von Lösungsmittelresten befreit. Das erhaltene rohe Allyl-(8-benzyl-1,4-dioxaspiro[4.5]dec-8-yl)methylamin (5,41 g) wurde ohne weitere Aufreinigung für 24 Stunden mit einem Gemisch aus 13 ml konz. Salzsäure (32 Massenprozent) und 7,5 ml Wasser bei Raumtemperatur gerührt. Anschließend wurde die Reaktionsmischung zunächst dreimal mit je 50 ml Diethylether gewaschen, dann unter Eiskühlung durch Zugabe von Natronlauge (32 Massenprozent) alkalisiert, dreimal mit je 100 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 3,55 g 4-(Allylmethylamino)-4-benzylcyclohexanon erhalten.
Zu 14,8 ml 1,0 molarer Phenethylmagnesiumchloridlösung in THF wurde unter Stickstoffatmosphäre eine Lösung von 3,50 g 4-(Allylmethylamino)-4-benzylcyclohexanon in 21 ml Tetrahydrofuran p.a. zugetropft und über Nacht bei Raumtemperatur gerührt. Zur Aufarbeitung wurden unter Eiskühlung 19 ml Ammoniumchloridlösung (20 Massenprozent) zugegeben, die Phasen getrennt, die wässrige Phase dreimal mit je 20 ml Diethylether extrahiert, die vereinigten organischen Phasen mit 20 ml Wasser gewaschen, dreimal mit je 20 ml verdünnter Salzsäure (5 Massenprozent) extrahiert, die vereinigten wässrigen Extrakte mit 20 ml Diethylether gewaschen, mit Ammoniaklösung (25 Massenprozent) auf ph 9 eingestellt, dreimal mit je 50 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (4,00 g braunes Harz) wurde mit Diethylether/Hexan (V/V = 1:1) an Kieselgel chromatographiert. Es wurden 2,04 g des unpolareren Diastereoisomers von 4-(Allylmethylamino)-4-benzyl-1-phenethylcyclohexanol erhaltenen, aus dem wie für Beispiel 4 beschrieben 807 mg des korrespondierenden Hydrochlorids hergestellt wurden.

**Beispiel 14**

4-Benzyl-4-dimethylamino-1-(3-fluorbenzyl)cyclohexanol Hydrochlorid

Unter Stickstoffatmosphäre wurden 757 mg Magnesium in 15 ml Diethylether p.a. gerührt und ca. ein Drittel der Lösung von 4,05 g 3-Fluorbenzylchlorid in 15 ml Diethylether p.a. zugegeben. Die restliche Lösung wurde nach dem Anspringen der Grignardbildung zügig zugetropft, nach beendeter Zugabe eine Stunde nachgerührt, anschließend 3,60 g 4-Benzyl-4-dimethylaminocyclohexanon, gelöst in 30 ml Diethylether p.a., zugetropft und die Reaktionsmischung über Nacht gerührt. Zur Aufarbeitung wurden unter Eiskühlung 31 ml Ammoniumchloridlösung (20 Massenprozent) zugegeben, die Phasen getrennt, die wässrige Phase zweimal mit je 40 ml Diethylether extrahiert, die vereinigten organischen Phasen mit 20 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (4,91 g gelbes Harz) wurde mit Diethylether/Hexan (V/V = 1:1) an Kieselgel chromatographiert. Es wurden 1,93 g des unpolareren Diastereoisomers von 4-Benzyl-4-dimethylamino-1-(3-fluorbenzyl)cyclohexanol als weißer Feststoff erhaltenen, aus dem wie für Beispiel 4 beschrieben 2,09 g des korrespondierenden Hydrochlorids hergestellt wurden.

**Beispiel 15**

4-Benzyl-4-dimethylamino-1-(4-fluorbenzyl)cyclohexanol Hydrochlorid

Unter Stickstoffatmosphäre wurden 757 mg Magnesium in 15 ml Diethylether p.a. gerührt und ca. ein Drittel der Lösung von 4,05 g 4-Fluorbenzylchlorid in 15 ml Diethylether p.a. zugegeben. Die restliche Lösung wurde nach dem Anspringen der Grignardbildung zügig zugetropft, nach beendeter Zugabe eine Stunde nachgerührt, anschließend 3,60

g 4-Benzyl-4-dimethylaminocyclohexanon, gelöst in 30 ml Diethylether p.a., zugetropft und die Reaktionsmischung über Nacht gerührt. Zur Aufarbeitung wurden unter Eiskühlung 31 ml Ammoniumchloridlösung (20 Massenprozent) zugegeben, die Phasen getrennt, die wässrige Phase zweimal mit je 40 ml Diethylether extrahiert, die vereinigten organischen Phasen mit 20 ml Wasser gewaschen, dreimal mit je 40 ml verdünnter Salzsäure (5 Massenprozent) extrahiert, die vereinigten wässrigen Extrakte mit 30 ml Diethylether gewaschen, mit Ammoniaklösung (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Das erhalte Rohprodukt (5,08 g gelbes Harz) wurde mit Diethylether/Hexan (V/V = 1:1) an Kieselgel chromatographiert. Es wurden 2,24 g des unpolareren Diastereoisomers von 4-Benzyl-4-dimethylamino-1-(4-fluorbenzyl)cyclohexanol als weißer Feststoff erhaltenen, aus dem wie für Beispiel 4 beschrieben 2,32 g des korrespondierenden Hydrochlorids hergestellt wurden.

## Beispiel 16

1-Benzyl-4-dimethylamino-4-(3-fluorbenzyl)cyclohexanol Hydrochlorid

Unter Stickstoffatmosphäre wurden 925 mg Magnesium in 19 ml Diethylether p.a. gerührt und ca. ein Drittel der Lösung von 4,95 g 3-Fluorbenzylchlorid in 19 ml Diethylether p.a. zugegeben. Die restliche Lösung wurde nach dem Anspringen der Grignardbildung zügig zugetropft, nach beendeter Zugabe eine Stunde nachgerührt, anschließend eine Lösung von 4,00 g 8-Dimethylamino-1,4-dioxaspiro[4.5]decan-8-carbonitril in 25 ml Diethylether p.a. zugetropft und die Reaktionsmischung über Nacht gerührt. Zur Aufarbeitung wurden unter Eiskühlung 29 ml Ammoniumchloridlösung (20 Massenprozent) zugegeben, die Phasen getrennt, die wässrige Phase zweimal mit je 50 ml Diethylether extrahiert, die vereinigten organischen Phasen nacheinander mit 20 ml Wasser und 20 ml gesättigter Natriumchloridlösung gewaschen. Das erhaltene rohe [8-(3-Fluorbenzyl)-1,4-dioxa-spiro[4.5]dec-8-yl]dimethylamin (5,75 g gelber Feststoff) wurde ohne weitere Aufreinigung für 24 Stunden mit einem Gemisch aus 14 ml konz. Salzsäure (32 Massenprozent) und 8 ml Wasser bei Raumtemperatur gerührt. Anschließend wurde die Reaktionsmischung zunächst dreimal mit je 30 ml Diethylether gewaschen, dann unter Eiskühlung durch Zugabe von wässrigem Ammoniak (25 Massenprozent) auf ph 9 eingestellt, dreimal mit je 40 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 4,71 g 4-Dimethylamino-4-(3-fluorbenzyl)cyclohexanon als gelber Feststoff erhalten. 4,67 g 4-Dimethylamino-4-(3-fluorbenzyl)cyclohexanon in Analogie zur Herstellung von 4-Dimethylamino-4-(4-fluorbenzyl)-1-phenethylcyclohexanol mit 1,0 molare Benzylmagnesiumchloridlösung in THF umgesetzt. Nach Chromatographie an Kieselgel wurde das erhaltene unpolarere Diastereoisomer von 1-Benzyl-4-dimethylamino-4-(3-fluorbenzyl)cyclohexanol wie für Beispiel 4 beschrieben in 261 mg des korrespondierenden Hydrochlorids überführt.

## Beispiel 17

4-Benzyl-1-phenethyl-4-pyrrolidin-1-ylcyclohexanol Hydrochlorid

Ein Gemisch aus 55 ml Wasser, 33 ml Salzsäure (32 Massenprozent), 50 ml Methanol, 127 ml Pyrrolidin, 50,0 g 1,4-Dioxaspiro[4.5]decan-8-on und 50,0 g Kaliumcyanid wurde für 65 Stunden bei Raumtemperatur gerührt. Die erhaltene hellbraune Suspension wurde viermal mit je 50 ml Diethylether extrahiert, die vereinigten Extrakte über Natriumsulfat getrocknet, filtriert, eingeengt und im Vakuum weitgehend von Lösungsmittelresten befreit. Es wurden 76,7 g 8-Pyrrolidin-1-yl-1,4-dioxaspiro[4.5]decan-8-carbonitril als hellbraune Flüssigkeit erhalten. Zu 127 ml 2,0 molarer Benzylmagnesiumchloridlösung in THF wurde unter Stickstoffatmosphäre eine Lösung von 40,0 g 8-Pyrrolidin-1-yl-1,4-dioxa-spiro[4.5]decan-8-carbonitril in 150 ml Tetrahydrofuran p.a. zugetropft und über Nacht bei Raumtemperatur gerührt. Zur Aufarbeitung wurden unter Eiskühlung 50 ml gesättigte Ammoniumchloridlösung zugegeben, die Phasen getrennt, die wässrige Phase dreimal mit je 50 ml Diethylether extrahiert, die vereinigten organischen Phasen über Natriumsulfat getrocknet, filtriert, eingeengt und im Vakuum weitgehend von Lösungsmittelresten befreit. Das erhaltene rohe 1-(8-Benzyl-1,4-dioxaspiro[4.5]dec-8-yl)pyrrolidin (54,0 g gelber Feststoff) wurde ohne weitere Aufreinigung für 24 Stunden mit einem Gemisch aus 128 ml konz. Salzsäure (32 Massenprozent) und 74 ml Wasser bei Raumtemperatur gerührt. Anschließend wurde die Reaktionsmischung zunächst zweimal mit je 50 ml Diethylether gewaschen, dann unter Eiskühlung durch Zugabe von Natronlauge (32 Massenprozent) alkalisiert, dreimal mit je 100 ml Dichlormethan extrahiert, die vereinigten Dichlormethan-Extrakte über Natriumsulfat getrocknet, filtriert, eingeengt und im Vakuum weitgehend von Lösungsmittelresten befreit. Es wurden 40,3 g 4-Benzyl-4-pyrrolidin-1-yl-cyclohexanon erhalten.

Zu 23,3 ml 1,0 molarer Phenethylmagnesiumchloridlösung in THF wurde unter Stickstoffatmosphäre und Eisbadkühlung eine Lösung von 4,00 g 4-Benzyl-4-pyrrolidin-1-yl-cyclohexanon in 40 ml Tetrahydrofuran p.a. zugetropft und über Nacht bei Raumtemperatur gerührt. Zur Aufarbeitung wurden unter Eiskühlung 25 ml Ammoniumchloridlösung (20 Massenprozent) zugegeben, die Phasen getrennt, die wässrige Phase dreimal mit je 50 ml Diethylether extrahiert,

die vereinigten organischen Phasen über Natriumsulfat getrocknet, filtriert, eingeengt und im Vakuum weitgehend von Lösungsmittelresten befreit. Das erhalte Rohprodukt (6,26 g braunes Öl) wurde mit Methanol/Ethylacetat (V/V = 1:1) an Kieselgel chromatographiert. Es wurden 2,48 g des unpolareren Diastereoisomers von 4-Benzyl-1-phenethyl-4-pyrrolidin-1-ylcyclohexanol erhaltenen, aus dem wie für Beispiel 4 beschrieben 1,98 g des korrespondierenden Hydrochlorids hergestellt wurden.

**Beispiel 18**

4-Benzyl-4-dimethylamino-1-(1-methyl-1H-indol-2-yl)cyclohexanol

Eine Lösung von N-Methylindol (1, 50 mg, 3,81 mmol) in trockenem THF (20 ml) wurde unter einem Argonstrom auf - 5 °C gekühlt. Danach wurde *tert*-Butyllithium (4,19 mmol, 2,47 ml einer 1,7 molaren Pentanlösung) so zugetropft, daß dabei eine Reaktionstemperatur von 0 °C nicht überschritten wurde. Nach beendeter Zugabe wurde die Reaktionsmischung weitere zwei Stunden bei 0 °C gerührt. Anschließend wurde eine Lösung von 4-Benzyl-4-dimethylaminocyclohexanon (3,88 mg, 3,81 mmol) in trockenem THF (7 ml) bei 0 °C zugetropft. Die Mischung wurde 15 Minuten bei 0 °C und anschließend vier Stunden bei Raumtemperatur gerührt. Das Reaktionsgemisch wurde mit gesättigter Ammoniumchloridlösung (20 ml) gequencht, die organische Phase abgetrennt und die wäßrige Phase viermal mit Dichlormethan (20 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V/V = 4:1). Es wurden 456 mg 4-Benzyl-4-dimethylamino-1-(1-methyl-1H-indol-2-yl)cyclohexanol mit einem Schmelzpunkt von 105 - 107 °C erhalten.

**Beispiel 19**

1-Benzo[b]thiophen-2-yl-4-benzyl-4-dimethylaminocyclohexanol

Eine Lösung von Benzo[b]thiophen (1, 50 mg, 3,73 mmol) in 20 ml trockenem THF wurde unter einem Argonstrom auf -5 °C gekühlt. Anschließend wurde *tert*-Butyllithium (4,47 mmol, 2,63 ml einer 1,7 molaren Pentanlösung) so zugetropft, daß dabei eine Reaktionstemperatur von 0 °C nicht überschritten wurde. Nach beendeter Zugabe wurde die Reaktionsmischung zwei Stunden bei 0 °C gerührt. Im Anschluß wurde eine Lösung von 4-Benzyl-4-dimethylaminocyclohexanon (3, 86 mg, 3,73 mmol) in trockenem THF (8 ml) bei 0 °C zugetropft. Die Mischung wurde 15 Minuten bei 0 °C und anschließend fünf Stunden bei Raumtemperatur gerührt. Das Reaktionsgemisch wurde mit gesättigter Ammoniumchloridlösung (30 ml) gequencht, die organische Phase abgetrennt und die wäßrige Phase viermal mit Dichlormethan (25 ml) extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V/V = 9:1). Es wurden 517 mg 1-Benzo[b]thiophen-2-yl-4-benzyl-4-dimethylaminocyclohexanol mit einem Schmelzpunkt von 128 - 131°C erhalten.

**Beispiel 20**

1-Benzo[b]thiophen-3-yl-4-benzyl-4-dimethylaminocyclohexanol

Eine Lösung von 3-Brom-1-benzo[b]thiophen (1, 90 mg, 4,22 mmol) in 30 ml trockenem Diethylether wurde unter einem Argonstrom auf -78 °C gekühlt. Danach wurde vorsichtig n-Butyllithium (5,07 mmol, 3,17 ml einer 15 massenprozentigen Hexanlösung) so zugetropft, daß dabei eine Reaktionstemperatur von -75 °C nicht überschritten wurde. Nach beendeter Zugabe wurde die Reaktionsmischung zwei Stunden bei -78 °C gerührt. Im Anschluß wurde eine Lösung von 4-Benzyl-4-dimethylaminocyclohexanon (977 mg, 4,22 mmol) in trockenem Diethylether (10 ml) bei -78 °C zugetropft. Die Mischung wurde vier Stunden bei -78 °C gerührt und anschließend langsam auf Raumtemperatur aufgetaut (ca. zwölf Stunden). Das Reaktionsgemisch wurde mit gesättigter Ammoniumchloridlösung (30 ml) gequencht, die organische Phase abgetrennt und die wäßrige Phase viermal mit Dichlormethan (25 ml) extrahiert. Die vereinigten organischen Phasen wurden über Natriumsulfat getrocknet, filtriert und das Lösungsmittel im Vakuum entfernt. Die Reinigung erfolgt mittels Flash-Chromatographie an Kieselgel mit Cyclohexan/Ethylacetat (V/V = 7:3). Es wurden 324 mg 1-Benzo[b]thiophen-3-yl-4-benzyl-4-dimethylaminocyclohexanol mit einem Schmelzpunkt von 158 - 160 °C erhalten.

**Beispiel 21**

1-Benzofuran-2-yl-4-benzyl-4-dimethylaminocyclohexanol

Eine Lösung von Benzo[b]furan (612 mg, 5,12 mmol) in trockenem THF (40 ml) wurde unter einem Argonstrom auf -8 °C gekühlt. Danach wurde *tert*-Butyllithium (6,22 mmol, 4,14 ml einer 1,5 molaren Pentanlösung) so zugetropft, dass eine Reaktionstemperatur von -5°C nicht überschritten wurde. Nach beendeter Zugabe wurde die Reaktionsmischung zwei Stunden bei -5°C gerührt. Im Anschluß wurde eine Lösung von 4-Benzyl-4-dimethylaminocyclohexanon (1,20 g, 5,18 mmol) in trockenem THF (10 ml) bei 0 °C zugetropft. Die Mischung wurde eine Stunde bei 0 °C und anschließend vier Tage bei Raumtemperatur gerührt. Das Reaktionsgemisch wurde mit gesättigter Ammoniumchloridlösung (20 ml) gequencht, die organische Phase abgetrennt und die wäßrige Phase mit viermal mit je 30 ml Dichlormethan extrahiert. Die vereinigten organischen Phasen wurden über Natriumsulfat getrocknet, filtriert und das Lösungsmittel im Vakuum entfernt. Die Reinigung erfolgte mittels Flash-Chromatographie an Kieselgel mit Cyclohexan/Ethylacetat (V/V = 8:2). Es wurden 380 mg 1-Benzofuran-2-yl-4-benzyl-4-dimethylaminocyclohexanol mit einem Schmelzpunkt von 121 - 124 °C erhalten.

| Beispiel | |
|---|---|
| Nr. | |
| 4 | 4-Benzyl-4-dimethylamino-1-phenethylcyclohexanol Hydrochlorid |
| 5 | 4-Dimethylamino-1,4-diphenethylcyclohexanol Hydrochlorid |
| 6 | 4-Benzyl-4-dimethylamino-1-[2-(2-fluorphenyl)ethyl]cyclohexanol Hydrochlorid |
| 7 | 4-Benzyl-4-dimethylamino-1-[2-(4-fluorphenyl)ethyl]cyclohexanol Hydrochlorid |
| 8 | 4-Dimethylamino-4-(2-fluorbenzyl)-1-phenethylcyclohexanol Hydrochlorid |
| 9 | 4-Dimethylamino-4-(3-fluorbenzyl)-1-phenethylcyclohexanol Hydrochlorid |
| 10 | 4-Dimethylamino-4-(4-fluorbenzyl)-1-phenethylcyclohexanol Hydrochlorid |
| 11 | 4-Benzyl-4-dimethylamino-1-[2-(3-fluorphenyl)ethyl]cyclohexanol Hydrochlorid |
| 12 | 4-Benzyl-4-dimethylamino-1-(2-fluorbenzyl)cyclohexanol Hydrochlorid |
| 13 | 4-(Allylmethylamino)-4-benzyl-1-phenethylcyclohexanol Hydrochlorid |
| 14 | 4-Benzyl-4-dimethylamino-1-(3-fluorbenzyl)cyclohexanol Hydrochlorid |
| 15 | 4-Benzyl-4-dimethylamino-1-(4-fluorbenzyl)cyclohexanol; Hydrochlorid |
| 16 | 1-Benzyl-4-dimethylamino-4-(3-fluorbenzyl)cyclohexanol Hydrochlorid |
| 17 | 4-Benzyl-1-phenethyl-4-pyrrolidin-1-ylcyclohexanol Hydrochlorid |
| 18 | 4-Benzyl-4-dimethylamino-1-(1-methyl-1 H-indol-2-yl)cyclohexanol |
| 19 | 1-Benzo[b]thiophen-2-yl-4-benzyl-4-dimethylaminocyclohexanol |
| 20 | 1-Benzo[b]thiophen-3-yl-4-benzyl-4-dimethylaminocyclohexanol |
| 21 | 1-Benzofuran-2-yl-4-benzyl-4-dimethylaminocyclohexanol |

**Patentansprüche**

**1.** Substituierte 4-Aminocyclohexanolderivate der allgemeinen Formel I,

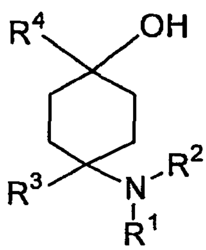

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; wobei $R^1$ und $R^2$ nicht beide H sein dürfen,

oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,

mit $R^5$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus $C_{3-8}$-Cycloalkyl, unsubstituiert oder einfach oder mehrfach substituiert; oder über eine gesättigte oder ungesättigte, unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^4$ ausgewählt ist aus $C_{3-8}$-Cycloalkyl, Aryl oder Heteroaryl, jeweils unsubstituiert oder einfach oder mehrfach substituiert; $-CHR^6R^7$, $-CHR^6-CH^2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder-$C(Y)-CH_2-CH_2-CH_2R^7$; oder-$R^8$-L-$R^9$

mit Y = O, S oder $H_2$,

mit $R^6$ ausgewählt aus

H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;

und mit R7 ausgewählt aus

H; $C_{3-8}$-Cycloalkyl, Aryl oder Heteroaryl, jeweils unsubstituiert oder einfach oder mehrfach substituiert,

mit $R^8$ ausgewählt aus

Aryl oder Heteroaryl, jeweils unsubstituiert oder einfach oder mehrfach substituiert,

mit L ausgewählt aus

$$-C(O)-NH-, -NH-C(O)-, -C(O)-O-, -O-C(O)-, -O-, -S- \text{ oder } -S(O)_2-$$

mit $R^9$ ausgewählt aus

Aryl oder Heteroaryl, jeweils unsubstituiert oder einfach oder mehrfach substituiert,

wobei "substituiert" im Zusammenhang mit Alkyl und Cycloalkyl bedeutet, dass mindestens ein (gegebenenfalls auch mehrere) Wasserstoffrest(e) durch F, Cl, Br, I, $NH_2$, SH oder OH ersetzt ist oder sind

und "substituiert" im Zusammenhang mit Aryl und Heteroaryl bedeutet, dass ein oder mehrere Wasserstoffreste durch $R^{82}$, $OR^{82}$ einem Halogen, einem $CF_3$, einem CN, einem $NO_2$ einem $NR^{83}R^{84}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen ersetzt ist,

wobei der Rest $R^{82}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen,

die Reste $R^{83}$ und $R^{84}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{83}$ und $R^{84}$ zusammen $CH_2CH_2OCH_2CH_2$, $CH_2CH_2NR^{85}CH_2CH_2$ oder $(CH_2)_{3-6}$ bedeuten, und

der Rest $R^{85}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen. gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

**2.** Substituierte 4-Aminocyclohexanolderivate gemäß Anspruch 1, **dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert; wobei $R^1$ und $R^2$ nicht beide H sein dürfen, oder die Reste $R^1$ und $R^2$ zusammen einen Ring bilden und $CH_2CH_2OCH_2CH_2$, $CH_2CH_2NR^5CH_2CH_2$ oder $(CH_2)_{3-6}$ bedeuten,
mit $R^5$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen, oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**3.** Substituierte 4-Aminocyclohexanolderivate gemäß einem der Ansprüche 1 oder 2, **dadurch gekennzeichnet, daß**
$R^3$ ausgewählt ist aus $C_{5-6}$-Cycloalkyl, unsubstituiert oder einfach oder mehrfach substituiert; oder über eine gesättigte, unverzweigte $C_{1-2}$-AlkylGruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;
insbesondere
$R^3$ ausgewählt ist aus über eine gesättigte, unverzweigte $C_{1-2}$-AlkylGruppe gebundenem Phenyl, Pyridyl, Furyl oder Thiophenyl, jeweils unsubstituiert oder einfach oder mehrfach substituiert.

**4.** Substituierte 4-Aminocyclohexanolderivate gemäß einem der Ansprüche 1 bis 3, **dadurch gekennzeichnet, daß**
$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$
vorzugsweise
$R^4$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; oder $-R^8-L-R^9$
insbesondere
$R^4$ ausgewählt ist aus Cyclopentyl, Cyclohexyl, Cycloheptyl, Cyclooctyl, Anthracenyl, Indolyl, Naphthyl, Benzothiazolyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert; oder $-R^8-L-R^9$.

**5.** Substituierte 4-Aminocyclohexanolderinate gemäß Anspruch 4, **dadurch gekennzeichnet, daß**
$R^8$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
L ausgewählt aus

-C(O)-NH-, -NH-C(O)-, -C(O)-O-, -O-C(O)-, -O-, -S- oder $-S(O)_2-$,

und/oder $R^9$ ausgewählt ist aus
Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,
vorzugsweise
$R^8$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,
L ausgewählt aus

-C(O)-NH-, -NH-C(O)-, -C(O)-O-, -O-C(O)- oder $-S(O)_2-$,

und/oder $R^9$ ausgewählt ist aus
Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert
insbesondere
$R^8$ ausgewählt ist aus
Indolyl, unubstituiert,
L ausgewählt aus

$-S(O)_2-$

und $R^9$ ausgewählt ist aus
Phenyl unsubstituiert.

6. Substituierte 4-Aminocyclohexanolderivate gemäß einem der Ansprüche 1 bis 3, **dadurch gekennzeichnet, daß**
$R^4$ ausgewählt ist aus $-CHR^6R^7$, $-CHR^6-\ CH_2R^7$, $-CHR^6-CH_2CH_2R^7$, $-\ CHR^6-CH_2-CH_2-CH_2R^7$, $-C(Y)R^7$, $-C(Y)-CH_2R^7$, $-C(Y)-CH_2-CH_2R^7$ oder-$C(Y)-CH_2CH_2-CH_2R^7$
mit Y = O, S oder $H_2$,
vorzugsweise
$R^4$ ausgewählt ist aus $-CHR^6R^7$, $-CHR^6-\ CH_2R^7$, $-CHR^6-CH_2-CH_2R^7$,$-C(Y)R^7$, $-C(Y)-CH_2R^7$ oder $-C(Y)-CH_2CH_2R^7$
mit Y = O oder S,
insbesondere
$R^4$ ausgewählt ist aus $-CHR^6R^7$, $-CHR^6-\ CH_2R^7$, $-C(Y)R^7$ oder $-C(Y)-CH_2R^7$
mit Y=O.

7. Substituierte 4-Aminocyclohexanolderivate gemäß Anspruch 6, **dadurch gekennzeichnet, daß**
$R^6$ ausgewählt ist aus
H, $C_{1-4}$-Alkyl, gesättigt oder ungesättigt, verzweigt oder unverzweigt, einfach oder mehrfach substituiert oder unsubstituiert; oder C(O)O-$C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert;
insbesondere
H, $CH_3$ und $C_2H_5$.

8. Substituierte 4-Aminocyclohexanolderivate gemäß Anspruch 6, **dadurch gekennzeichnet, daß**
$R^7$ ausgewählt ist aus $C_{3-8}$-Cycloalkyl, Aryl oder Heteroaryl, jeweils unsubstituiert oder einfach oder mehrfach substituiert;
vorzugsweise
$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**9.** Substituierte 4-Aminocyclohexanolderivate gemäß einem der Ansprüche 1 bis 8, **dadurch gekennzeichnet, daß** sie ausgewählt sind aus der folgenden Gruppe:

- 4-Benzyl-4-dimethylamino-1-phenethylcyclohexanol sowie dem entsprechenden Hydrochlorid,
- 4-Dimethylamino-1,4-diphenethylcyclohexanol sowie dem entsprechenden Hydrochlorid,
- 4-Benzyl-4-dimethylamino-1-[2-(2-fluorphenyl)ethyl]cyclohexanol sowie dem entsprechenden Hydrochlorid,
- 4-Benzyl-4-dimethylamino-1-[2-(4-fluorphenyl)ethyl]cyclohexanol sowie dem entsprechenden Hydrochlorid,
- 4-Dimethylamino-4-(2-fluorbenzyl)-1-phenethylcyclohexanol sowie dem entsprechenden Hydrochlorid,
- 4-Dimethylamino-4-(3-fluorbenzyl)-1-phenethylcyclohexanol sowie dem entsprechenden Hydrochlorid,
- 4-Dimethylamino-4-(4-fluorbenzyl)-1-phenethylcyclohexanol sowie dem entsprechenden Hydrochlorid,
- 4-Benzyl-4-dimethylamino-1-[2-(3-fluorphenyl)ethyl]cyclohexanol sowie dem entsprechenden Hydrochlorid,
- 4-Benzyl-4-dimethylamino-1-(2-fluorbenzyl)cyclohexanol sowie dem entsprechenden Hydrochlorid,
- 4-(Allylmethylamino)-4-benzyl-1-phenethylcyclohexanol sowie dem entsprechenden Hydrochlorid,
- 4-Benzyl-4-dimethylamino-1-(3-fluorbenzyl)cyclohexanol sowie dem entsprechenden Hydrochlorid,
- 4-Benzyl-4-dimethylamino-1-(4-fluorbenzyl)cyclohexanol sowie dem entsprechenden Hydrochlorid,
- 1-Benzyl-4-dimethylamino-4-(3-fluorbenzyl)cyclohexanol sowie dem entsprechenden Hydrochlorid oder
- 4-Benzyl-1-phenethyl-4-pyrrolidin-1-ylcyclohexanol sowie dem entsprechenden Hydrochlorid
- 4-Benzyl-4-dimethylamino-1-(1-methyl-1H-indol-2-yl)cyclohexanol
- 1-Benzo[b]thiophen-2-yl-4-benzyl-4-dimethylaminocyclohexanol
- 1-Benzo[b]thiophen-3-yl-4-benzyl-4-dimethylaminocyclohexanol
- 1-Benzofuran-2-yl-4-benzyl-4-dimethylamino-cyclohexanol

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

**10.** Arzneimittel enthaltend wenigstens ein substituiertes 4-Aminocyclohexanol-derivat gemäß einem der Ansprüche 1 bis 9 gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form der Säuren oder der Basen oder in Form der Salze, insbesondere der physiologisch verträglichen Salze, oder in Form der Solvate, insbesondere der Hydrate; sowie gegebenenfalls geeignete Zusatz- und/oder Hilfsstoffe und/oder gegebenenfalls weitere Wirkstoffe.

**11.** Arzneimittel gemäß Anspruch 10, **dadurch gekennzeichnet, daß** das Arzneimittel neben wenigstens einem substituierten 4-Aminocyclohexanolderivat noch ein Opioid, vorzugsweise ein starkes Opioid, insbesondere Morphin, oder ein Anesthetikum, vorzugsweise Hexobarbital oder Halothan, enthält.

**12.** Verwendung eines substituierten 4-Aminocyclohexanolderivats gemäß einem der Ansprüche 1 bis 9 gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, oder in Form seiner Solvate, insbesondere der Hydrate; zur Herstellung eines Arzneimittels zur Behandlung von Schmerz, insbesondere von akutem, viszeralem, neuropathischem oder chronischem Schmerz.

**13.** Verwendung eines substituierten 4-Aminocyclohexanolderivats gemäß einem der Ansprüche 1 bis 9 gegebenenfalls in Form seiner Racemate, seiner reinen Stereoisomeren, insbesondere Enantiomeren oder Diastereomeren, oder

in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, oder in Form seiner Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/ oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum, Antitussivum oder Anesthetikum bzw. zur Coadministration bei Behandlung mit einem opioiden Analgetikum oder mit einem Anesthetikum, zur Diurese oder Antinatriurese und/oder Anxiolyse.

**14.** Verfahren zur Herstellung eines substituierten 4-Aminocyclohexanolderivats gemäß einem der Ansprüche 1 bis 9 mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

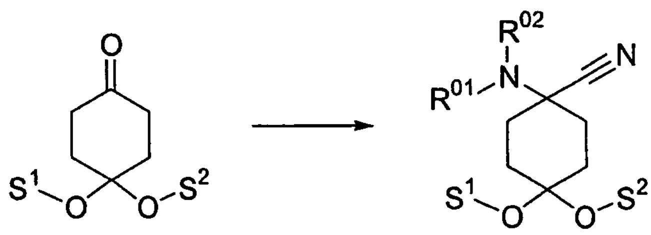

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IVa entsteht;

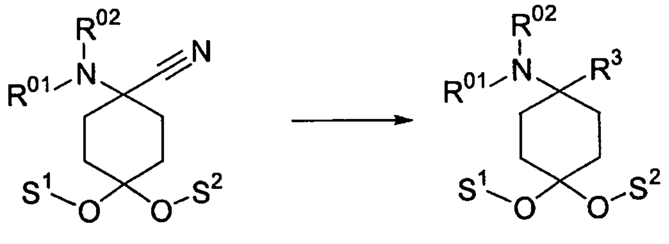

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa gemäß Formel III werden die Schutzgruppen $S^1$ und $S^2$ abgespalten,

so daß ein 4-substituiertes 4-Aminocyclohexanonderivat gemäß Formel IV entsteht;

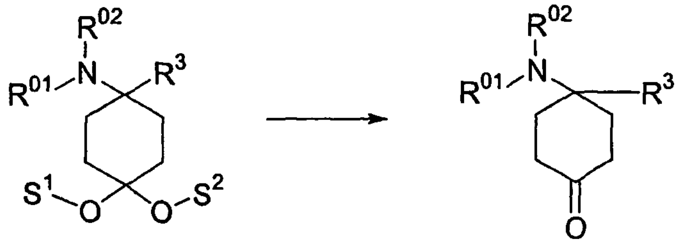

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IV mit metallorganischen Reagenzien, bevorzugt Grignard- oder Organolithiumreagenzien, der Formel Metall-$R^3$ umgesetzt, so daß eine Verbindung gemäß Formel V entsteht;

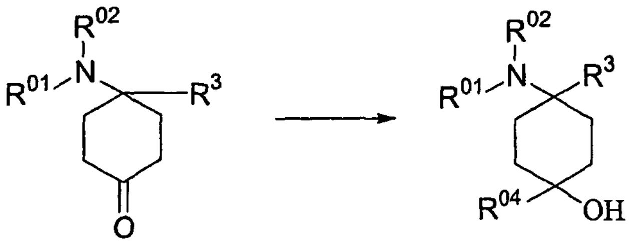

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$ $R^4$ und $R^5$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{02}$ zusammen einen Ring bilden und
$CH_2CH_2OCH_2CH_2$, $CH_2CH_2NR^{05}$ $CH_2CH_2$ oder $(CH_2)_{3-6}$ bedeuten,
mit $R^{05}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^{04}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ oder $-C(Y)-CH_2-CH_2-CH_2R^7$; oder $-R^8-L-R^9$
mit Y = O, S oder $H_2$,
mit $R^6$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit R7 ausgewählt aus
H; $C_{3-8}$-Cycloalkyl, Aryl oder Heteroaryl, jeweils unsubstituiert oder einfach oder mehrfach substituiert,
mit $R^8$ ausgewählt aus
Aryl oder Heteroaryl, jeweils unsubstituiert oder einfach oder mehrfach substituiert,
mit L ausgewählt aus

$$-C(O)-NH-, -NH-C(O)-, -C(O)-O-, -O-C(O)-, -O-, -S- \text{ oder } -S(O)_2-$$

mit $R^9$ ausgewählt aus
Aryl oder Heteroaryl, jeweils unsubstituiert oder einfach oder mehrfach substituiert,
und $S^1$ und $S^2$ unabhängig voneinander ausgewählt sind aus Schutzgruppen oder zusammen eine Schutzgruppe bedeuten, vorzugsweise Monoacetal.

**15.** Verfahren zur Herstellung eines substituierten 4-Aminocyclohexanolderivats gemäß Anspruch 14, **dadurch gekennzeichnet, daß** die Schutzgruppen am H bei $R^{01}$, $R^{02}$, $R^{04}$ und/oder $R^{05}$ ausgewählt sind aus Alkyl, Benzyl oder Carbamaten, beispielsweise FMOC, Z oder Boc.

## Claims

**1.** Substituted 4-aminocyclohexanol derivatives of the general formula I

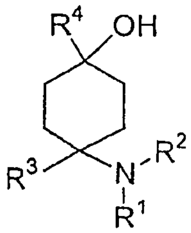

I

wherein
$R^1$ and $R^2$ independently of one another are chosen from H; $C_{1-8}$-alkyl or $C_{3-8}$-cycloalkyl, in each case saturated or unsaturated, branched or unbranched, mono- or polysubstituted or unsubstituted; aryl-, or heteroaryl, in each case mono- or polysubstituted or unsubstituted; or aryl, $C_{3-8}$-cycloalkyl or heteroaryl bonded via $C_{1-3}$-alkylene and in each case mono- or polysubstituted or unsubstituted; wherein $R^1$ and $R^2$ may not both be H,
or the radicals $R^1$ and $R^2$ together form a ring and denote $CH_2CH_2OCH_2CH_2$, $CH_2CH_2NR^5CH_2CH_2$ or $(CH_2)_{3-6}$,
where $R^5$ is chosen from H; $C_{1-8}$-alkyl or $C_{3-8}$-cycloalkyl, in each case saturated or unsaturated, branched or unbranched, mono- or polysubstituted or unsubstituted; aryl or heteroaryl, in each case mono- or polysubstituted or unsubstituted; or aryl, $C_{3-8}$-cycloalkyl or heteroaryl bonded via $C_{1-3}$- alkylene and in each case mono- or polysubstituted or unsubstituted;
$R^3$ is chosen from $C_{3-8}$-cycloalkyl, unsubstituted or mono- or polysubstituted; or aryl, $C_{3-8}$-cycloalkyl or heteroaryl bonded via a saturated or unsaturated, unbranched, substituted or unsubstituted $C_{1-4}$-alkyl group and in each case unsubstituted or mono- or polysubstituted;
$R^4$ is chosen from $C_{3-8}$-cycloalkyl, aryl or heteroaryl, in each case unsubstituted or mono- or polysubstituted; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or $-R^8-L-R^9$
where Y = O, S or $H_2$,

where $R^6$ is chosen from H, $C_{1-7}$-alkyl, saturated or unsaturated, branched or unbranched, mono- or polysubstituted or unsubstituted; or C(O)O-$C_{1-6}$-alkyl, saturated or unsaturated, branched or unbranched, mono- or polysubstituted or unsubstituted; and where $R^7$ is chosen from H; $C_{3-8}$-cycloalkyl, aryl or heteroaryl, in each case unsubstituted or mono- or polysubstituted, where $R^8$ is chosen from aryl or heteroaryl, in each case unsubstituted or mono- or polysubstituted, where L is chosen from

$$-C(O)-NH-, -NH-C(O)-, -C(O)-O-, -O- C(O)-, -O- , -S- \text{ or } -S(O)_2-$$

where $R^9$ is chosen from aryl or heteroaryl, in each case unsubstituted or mono- or polysubstituted, wherein "substituted" in connection with alkyl and cycloalkyl denotes that at least one (optionally also several) hydrogen radical(s) is/are replaced by F, Cl, Br, I, $NH_2$, SH or OH, and "substituted" in connection with aryl and heteroaryl denotes that one or more hydrogen radical is/are replaced by $R^{82}$, $OR^{82}$, a halogen, a $CF_3$, a CN, an $NO_2$, an $NR^{83}R^{84}$, a $C_{1-6}$-alkyl (saturated), a $C_{1-6}$-alkoxy, a $C_{3-8}$-cycloalkoxy, a $C_{3-8}$-cycloalkyl or a $C_{2-6}$-alkylene, wherein the radical $R^{82}$ denotes H, a $C_{1-10}$-alkyl, preferably a $C_{1-6}$-alkyl, an aryl or heteroaryl, or an aryl or heteroaryl radical bonded via $C_{1-3}$-alkyl, saturated or unsaturated, or a $C_{1-3}$-alkylene group, wherein these aryl and heteroaryl radicals may not themselves be substituted by aryl or heteroaryl radicals, the radicals $R^{83}$ and $R^{84}$, which are identical or different, denote H, a $C_{1-10}$-alkyl, preferably a $C_{1-6}$-alkyl, an aryl, a heteroaryl, or an aryl or heteroaryl radical bonded via $C_{1-3}$-alkyl, saturated or unsaturated, or a $C_{1-3}$-alkylene group, wherein these aryl and heteroaryl radicals may not themselves be substituted by aryl or heteroaryl radicals, or the radicals $R^{83}$ and $R^{84}$ together denote $CH_2CH_2OCH_2CH_2$, $CH_2CH_2NR^{85}CH_2CH_2$ or $(CH_2)_{3-6}$, and the radical $R^{85}$ denotes H, a $C_{1-10}$-alkyl, preferably a $C_{1-6}$-alkyl, an aryl or heteroaryl radical, or an aryl or heteroaryl radical bonded via $C_{1-3}$-alkyl, saturated or unsaturated, or a $C_{1-3}$-alkylene group, wherein these aryl and heteroaryl radicals may not themselves be substituted by aryl or heteroaryl radicals, optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates.

2. Substituted 4-aminocyclohexanol derivatives according to claim 1, **characterized in that** $R^1$ and $R^2$ independently of one another are chosen from H; $C_{1-8}$-alkyl, saturated or unsaturated, branched or unbranched, mono- or polysubstituted or unsubstituted; where $R^1$ and $R^2$ may not both be H, or the radicals $R^1$ and $R^2$ together form a ring and denote $CH_2CH_2OCH_2CH_2$, $CH_2CH_2NR^5CH_2CH_2$ or $(CH_2)_{3-6}$, where $R^5$ is chosen from H; $C_{1-8}$-alkyl, saturated or unsaturated, branched or unbranched, mono- or polysubstituted or unsubstituted, preferably $R^1$ and $R^2$ independently of one another are chosen from H; $C_{1-4}$-alkyl, saturated or unsaturated, branched or unbranched, mono- or polysubstituted or unsubstituted; where $R^1$ and $R^2$ may not both be H, or the radicals $R^1$ and $R^2$ together form a ring and denote $(CH_2)_{4-5}$, in particular $R^1$ and $R^2$ independently of one another are chosen from methyl or ethyl or the radicals $R^1$ and $R^2$ together form a ring and denote $(CH_2)_5$.

3. Substituted 4-aminocyclohexanol derivatives according to one of claims 1 and 2, **characterized in that** $R^3$ is chosen from $C_{5-6}$-cycloalkyl, unsubstituted or mono- or polysubstituted; or $C_{5-6}$-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl bonded via a saturated, unbranched $C_{1-2}$-alkyl group and in each case unsubstituted or mono- or polysubstituted; in particular $R^3$ is chosen from phenyl, pyridyl, furyl or thiophenyl bonded via a saturated, unbranched $C_{1-2}$-alkyl group and in each case unsubstituted or mono- or polysubstituted.

4. Substituted 4-aminocyclohexanol derivatives according to one of claims 1 to 3, **characterized in that** $R^4$ is chosen

from $C_{3-8}$-cycloalkyl, aryl or heteroaryl, in each case unsubstituted or mono- or polysubstituted; or $-R^8$-L-$R^9$
preferably
$R^4$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo [1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted; or $-R^8$-L-$R^9$
in particular
$R^4$ is chosen from cyclopentyl, cyclohexyl, cycloheptyl, cyclooctyl, anthracenyl, indolyl, naphthyl, benzothiazolyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted; or $-R^8$-L-$R^9$.

5. Substituted 4-aminocyclohexanol derivatives according to claim 4, **characterized in that**
$R^8$ is chosen from
indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl, in each case unsubstituted or mono- or polysubstituted,
L is chosen from

-C (O) -NH-, -NH-C(O)-, -C (O) -O-, -O-

C(O)-, -O-, -S- OR $-S(O)_2$-,

and/or $R^9$ is chosen from
indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl, in each case unsubstituted or mono- or polysubstituted,
preferably
$R^8$ is chosen from
indolyl, benzothiophenyl, phenyl, thiophenyl, furyl, pyridyl, pyrrolyl, pyrazinyl or pyrimidyl, in each case unsubstituted or mono- or polysubstituted,
L is chosen from -C(O)-NH-, -NH-C(O)-, -C(O)-O-, -O-C(O)- or- $S(O)_2$-,
and/or $R^9$ is chosen from
indolyl, benzothiophenyl, phenyl, thiophenyl, furyl, pyridyl, pyrrolyl, pyrazinyl or pyrimidyl, in each case unsubstituted or mono- or polysubstituted,
in particular
$R^8$ is chosen from
indolyl, unbstituted,
L is chosen from

$-S(O)_2$-

and $R^9$ is chosen from
phenyl, unsubstituted.

6. Substituted 4-aminocyclohexanol derivatives according to one of claims 1 to 3, **characterized in that**
$R^4$ is chosen from $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$
where Y = O, S or $H_2$,
preferably
$R^4$ is chosen from $-CHR^6R^7$, $-CHR^6-CH_2R^7$, $-CHR^6-CH_2-CH_2R^7$, $-C(Y)R^7$, $-C(Y)-CH_2R^7$ or $-C(Y)-CH_2-CH_2R^7$
where Y = O or S,
in particular

$R^4$ is chosen from $-CHR^6R^7$, $-CHR^6-CH_2R^7$, $-C(Y)R^7$ or $-C(Y)-CH_2R^7$
where Y = O.

**7.** Substituted 4-aminocyclohexanol derivatives according to claim 6, **characterized in that**
$R^6$ is chosen from
H, $C_{1-4}$-alkyl, saturated or unsaturated, branched or unbranched, mono- or polysubstituted or-unsubstituted; or C (O)O-$C_{1-4}$-alkyl, saturated or unsaturated, branched or unbranched, mono- or polysubstituted or unsubstituted;
preferably
H, $C_{1-4}$-alkyl, saturated or unsaturated, branched or unbranched, mono- or polysubstituted or unsubstituted;
in particular
H, $CH_3$ and $C_2H_5$.

**8.** Substituted 4-aminocyclohexanol derivatives according to claim 6, **characterized in that**
$R^7$ is chosen from $C_{3-8}$-cycloalkyl, aryl or heteroaryl, in each case unsubstituted or mono- or polysubstituted;
preferably
$R^7$ is chosen from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo [1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, in each case unsubstituted or mono- or polysubstituted;
in particular
$R^7$ is chosen from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, in each case unsubstituted or mono- or polysubstituted.

**9.** Substituted 4-aminocyclohexanol derivatives according to one of claims 1 to 8, **characterized in that** they are chosen from the following group:

- 4-benzyl-4-dimethylamino-1-phenethylcyclohexanol and the corresponding hydrochloride,
- 4-dimethylamino-1,4-diphenethylcyclohexanol and the corresponding hydrochloride,
- 4-benzyl-4-dimethylamino-1-[2-(2-fluorophenyl)ethyl]cyclohexanol and the corresponding hydrochloride,
- 4-benzyl-4-dimethylamino-1-[2-(4-fluorophenyl)ethyl]cyclohexanol and the corresponding hydrochloride,
- 4-dimethylamino-4-(2-fluorobenzyl)-1-phenethylcyclohexanol and the corresponding hydrochloride,
- 4-dimethylamino-4-(3-fluorobenzyl)-1-phenethylcyclohexanol and the corresponding hydrochloride,
- 4-dimethylamino-4-(4-fluorobenzyl)-1-phenethylcyclohexanol and the corresponding hydrochloride,
- 4-benzyl-4-dimethylamino-1-[2-(3-fluorophenyl)ethyl]cyclohexanol and the corresponding hydrochloride
- 4-benzyl-4-dimethylamino-1-(2-fluorobenzyl)cyclohexanol and the corresponding hydrochloride,
- 4-(allylmethylamino)-4-benzyl-1-phenethylcyclohexanol and the corresponding hydrochloride,
- 4-benzyl-4-dimethylamino-1-(3-fluorobenzyl)cyclohexanol and the corresponding hydrochloride,
- 4-benzyl-4-dimethylamino-1-(4-fluorobenzyl)cyclohexanol and the corresponding hydrochloride,
- 1-benzyl-4-dimethylamino-4-(3-fluorobenzyl)cyclohexanol and the corresponding hydrochloride or
- 4-benzyl-1-phenethyl-4-pyrrolidin-1-ylcyclohexanol and the corresponding hydrochloride
- 4-benzyl-4-dimethylamino-1-(1-methyl-1H-indol-2-yl)cyclohexanol
- 1-benzo[b]thiophen-2-yl-4-benzyl-4-dimethylaminocyclohexanol
- 1-benzo[b]thiophen-3-yl-4-benzyl-4-dimethylaminocyclohexanol
- 1-benzofuran-2-yl-4-benzyl-4-dimethylaminocyclohexanol

optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates.

**10.** Medicament comprising at least one substituted 4-aminocyclohexanol derivative according to one of claims 1 to 9, optionally in the form of its racemate, its pure stereoisomers, in particular enantiomers or diastereomers, or in the form of mixtures of the stereoisomers, in particular the enantiomers or diastereomers, in any desired mixing ratio; in the form shown or in the form of the acids or the bases or in the form of the salts, in particular the physiologically acceptable salts, or in the form of the solvates, in particular the hydrates; and optionally suitable additives and/or

auxiliary substances and/or optionally further active compounds.

**11.** Medicament according to claim 10, **characterized in that** in addition to at least one substituted 4-aminocyclohexanol derivative, the medicament also contains an opioid, preferably a potent opioid, in particular morphine, or an anaesthetic, preferably hexobarbital or halothane.

**12.** Use of a substituted 4-aminocyclohexanol derivative according to one of claims 1 to 9, optionally in the form of its racemates, its pure stereoisomers, in particular enantiomers or diastereomers, or in the form of mixtures of the stereoisomers, in particular the enantiomers or diastereomers, in any desired mixing ratio; in the form shown or in the form of its acids or its bases or in the form of its salts, in particular the physiologically acceptable salts, or in the form of its solvates, in particular the hydrates; for the preparation of a medicament for treatment of pain, in particular acute, visceral, neuropathic or chronic pain.

**13.** Use of a substituted 4-aminocyclohexanol derivative according to one of claims 1 to 9, optionally in the form of its racemates, its pure stereoisomers, in particular enantiomers or diastereomers, or in the form of mixtures of the stereoisomers, in particular the enantiomers or diastereomers, in any desired mixing ratio; in the form shown or in the form of its acids or its bases or in the form of its salts, in particular the physiologically acceptable salts, or in the form of its solvates, in particular the hydrates; for the preparation of a medicament for treatment of anxiety states, of stress and stress-associated syndromes, depressions, epilepsy, Alzheimer's disease, senile dementia, general cognitive dysfunctions, learning and memory difficulties (as a nootropic), withdrawal symptoms, alcohol and/or drug and/or medicament abuse and/or dependency, sexual dysfunctions, cardiovascular diseases, hypotension, hypertension, tinnitus, pruritus, migraine, impaired hearing, deficient intestinal motility, impaired food intake, anorexia, obesity, locomotor disorders, diarrhoea, cachexia, urinary incontinence or as a muscle relaxant, anticonvulsive, antitussive or anaesthetic or for co-administration in treatment with an opioid analgesic or with an anaesthetic, for diuresis or antinatriuresis and/or anxiolysis.

**14.** Process for the preparation of a substituted 4-aminocyclohexanol derivative according to one of claims 1 to 9 with the following steps:

a. a cyclohexane-1,4-dione, protected with the groups $S^1$ and $S^2$, according to formula II is reacted with a cyanide, preferably potassium cyanide, in the presence of a compound of the formula $HNR^{01}R^{02}$ to give a protected N-substituted 1-amino-4-oxo-cyclohexanecarbonitrile derivative according to formula III;

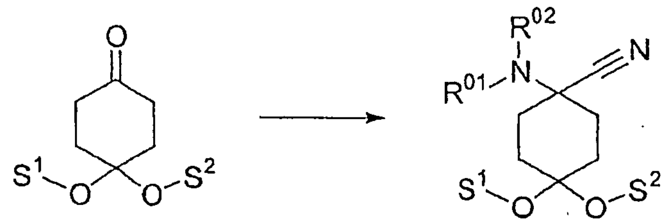

II III

optionally acylation, alkylation or sulfonation is then carried out in any desired sequence and optionally repeatedly, and/or in the case of compounds where $R^{01}$ and/or $R^{02}$ and/or $R^{06}$ = H protected with a protective group, at least once a protective group is split off and optionally acylation, alkylation or sulfonation is carried out, and/or in the case of compounds where $R^{01}$ and/or $R^{02}$ and/or $R^{06}$ = H, at least once a protective group is introduced and optionally acylation, alkylation or sulfonation is carried out,

b. the aminonitrile according to formula III is reacted with organometallic reagents, preferably Grignard or organolithium reagents, of the formula metal-$R^3$, so that a compound according to formula IVa is formed;

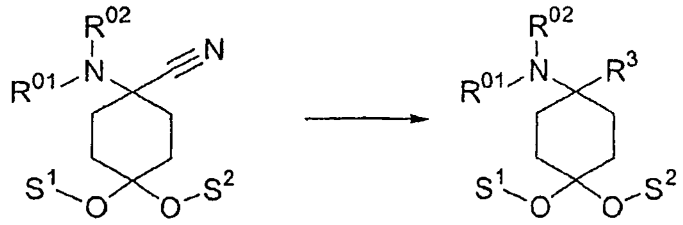

III IVa

optionally acylation, alkylation or sulfonation is then carried out in any desired sequence and optionally repeatedly, and/or in the case of compounds where $R^{01}$ and/or $R^{02}$ and/or $R^{06}$ = H protected with a protective group, at least once a protective group is split off and optionally acylation, alkylation or sulfonation is carried out, and/or in the case of compounds where $R^{01}$ and/or $R^{02}$ and/or $R^{06}$ = H, at least once a protective group is introduced and optionally acylation, alkylation or sulfonation is carried out,

c. on the compound according to formula IVa according to formula III, the protective groups $S^1$ and $S^2$ are split off so that a 4-substituted 4-aminocyclohexanone derivative according to formula IV is formed;

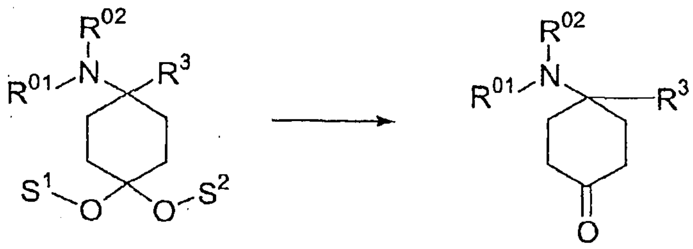

IVa IV

optionally acylation, alkylation or sulfonation is then carried out in any desired sequence and optionally repeatedly, and/or in the case of compounds where $R^{01}$ and/or $R^{02}$ and/or $R^{06}$ = H protected with a protective group, at least once a protective group is split off and optionally acylation, alkylation or sulfonation is carried out, and/or in the case of compounds where $R^{01}$ and/or $R^{12}$ and/or $R^{06}$ = H, at least once a protective group is introduced and optionally acylation, alkylation or sulfonation is carried out,

d. the 4-substituted 4-aminocyclohexanone derivative according to formula IV is reacted with organometallic reagents, preferably Grignard or organolithium reagents, of the formula metal-$R^3$ so that a compound according to formula V is formed;

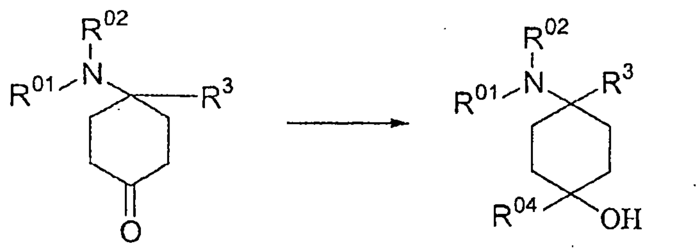

IV V

optionally acylation, alkylation or sulfonation is then carried out in any desired sequence and optionally repeatedly, and/or in the case of compounds where $R^{01}$ and/or $R^{02}$ and/or $R^{04}$ and/or $R^{05}$ and/or $R^{06}$ = H protected with a protective group, at least once a protective group is split off and optionally acylation, alkylation or sulfonation

is carried out, and/or in the case of compounds where $R^{01}$ and/or $R^{02}$ and/or $R^{04}$ and/or $R^{05}$ and/or $R^{06}$ = H, at least once a protective group is introduced and optionally acylation, alkylation or sulfonation is carried out, until a compound according to formula I is formed,

wherein $R^1$, $R^2$, $R^3$, $R^4$ and $R^5$ have the meaning given in claim 1
and
$R^{01}$ and $R^{02}$ independently of one another are chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl or heteroaryl, in each case mono- or polysubstituted or unsubstituted; or aryl, $C_{3-8}$-cycloalkyl or heteroaryl bonded via $C_{1-3}$-alkylene and in each case mono- or polysubstituted or unsubstituted;
or the radicals $R^{01}$ and $R^{02}$ together form a ring and denote $CH_2CH_2OCH_2$ $CH_2CH_2NR^{05}CH_2CH_2$ or $(CH_2)_{3-6}$,
where $R^{05}$ is chosen from H; H provided with a protective group; $C_{1-8}$-alkyl or $C_{3-8}$-cycloalkyl, in each case saturated or unsaturated, branched or unbranched, mono- or polysubstituted or unsubstituted; aryl or heteroaryl, in each case mono- or polysubstituted or unsubstituted; or aryl, $C_{3-8}$-cycloalkyl or heteroaryl bonded via $C_{1-3}$-alkylene and in each case mono- or polysubstituted or unsubstituted;
$R^{04}$ is chosen from H, H provided with a protective group; $C_{3-8}$-cycloalkyl, aryl or heteroaryl, in each case unsubstituted or mono- or polysubstituted; $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ or $-C(Y)-CH_2-CH_2-CH_2R^7$; or $-R^8-L-R^9$
where Y = O, S or $H_2$,
where $R^6$ is chosen from
H, $C_{1-7}$-alkyl, saturated or unsaturated, branched or unbranched, mono- or polysubstituted or unsubstituted; or $C(O)O-C_{1-6}$-alkyl, saturated or unsaturated, branched or unbranched, mono- or polysubstituted or unsubstituted;
and where $R^7$ is chosen from
H; $C_{3-8}$-cycloalkyl, aryl or heteroaryl, in each case unsubstituted or mono- or polysubstituted,
where $R^8$ is chosen from
aryl or heteroaryl, in each case unsubstituted or mono- or polysubstituted,
where L is chosen from

-C(O)-NH-, -NH-C(O)-, -C(O)-O-, -O-

C(O)-, -O-, -S- or $-S(O)_2-$

where $R^9$ is chosen from
aryl or heteroaryl, in each case unsubstituted or mono- or polysubstituted,
and $S^1$ and $S^2$ independently of one another are chosen from protective groups or together denote a protective group, preferably monoacetal.

**15.** Process for the preparation of a substituted 4-aminocyclohexanol derivative according to claim 14, **characterized in that** the protective groups on H in $R^{01}$, $R^{02}$, $R^{04}$ and/or $R^{05}$ are chosen from alkyl, benzyl or carbamates, for example FMOC, Z or Boc.

## Revendications

**1.** Dérivés substitués de 4-aminocyclohexanol de formule générale I,

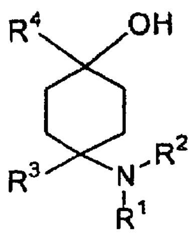

I

formule dans laquelle

$R^1$ et $R^2$ sont choisis, indépendamment l'un de l'autre, entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste hétéroaryle, cycloalkyle en $C_3$ à $C_8$ ou aryle lié par l'intermédiaire d'un radical alkylène en $C_1$ à $C_3$, chacun substitué une ou plusieurs fois ou non substitué ; $R^1$ et $R^2$ ne devant pas représenter tous deux H ;

ou bien les restes $R^1$ et $R^2$ forment ensemble un noyau et représentent un groupe $CH_2CH_2OCH_2CH_2$, $CH_2CH_2NR^5CH_2CH_2$ ou $(CH_2)_{3-6}$,

$R^5$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste hétéroaryle, cycloalkyle en $C_3$ à $C_8$ ou aryle lié par l'intermédiaire d'un radical alkylène en $C_1$ à $C_3$, chacun substitué une ou plusieurs fois ou non substitué ;

$R^3$ est choisi entre un reste cycloalkyle en $C_3$ à $C_8$ non substitué ou substitué une ou plusieurs fois ; ou un reste hétéroaryle, cycloalkyle en $C_3$ à $C_8$ ou aryle lié par l'intermédiaire d'un groupe alkyle en $C_1$ à $C_4$ saturé ou non saturé, non ramifié, substitué ou non substitué, chacun non substitué ou substitué une ou plusieurs fois ;

$R^4$ est choisi entre un reste cycloalkyle en $C_3$ à $C_8$, aryle ou hétéroaryle, chacun non substitué ou substitué une ou plusieurs fois ; un reste $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, $-C(Y)-CH_2R^7$, $-C(Y)-CH_2-CH_2R^7$ ou $-C(Y)-CH_2-CH_2-CH_2R^7$ ; ou $-R^8-L-R^9$

avec Y = O, S ou $H_2$,

$R^6$ étant choisi entre H, un reste alkyle en $C_1$ à $C_7$, saturé ou non saturé, ramifié ou non ramifié, substitué une ou plusieurs fois ou non substitué ; ou un reste C(O)O-(alkyle en $C_1$ à $C_6$), saturé ou non saturé, ramifié ou non ramifié, substitué une ou plusieurs fois ou non substitué ;

et $R^7$ étant choisi entre

H ; un reste cycloalkyle en $C_3$ à $C_8$, aryle ou hétéroaryle, chacun non substitué ou substitué une ou plusieurs fois,

$R^8$ étant choisi entre

un reste aryle ou hétéroaryle, chacun non substitué ou substitué une ou plusieurs fois,

L étant choisi entre

-C(O)-NH-, -NH-C(O)-, -C(O)-O-, -O-C(O)-, -O-, -Sou $-S(O)_2$,

$R^9$ étant choisi entre

un reste aryle ou hétéroaryle, chacun non substitué ou substitué une ou plusieurs fois,

le terme « substitué » en rapport avec alkyle et cycloalkyle signifiant qu'au moins un reste hydrogène (même plusieurs le cas échéant) est remplacé par F, Cl, Br, I, $NH_2$, SH ou OH

et le terme « substitué » en rapport avec aryle et hétéroaryle signifiant qu'un ou plusieurs restes hydrogène sont remplacés par $R^{82}$, $OR^{82}$, un halogène, un groupe $CF_3$, un groupe CN, un groupe $NO_2$ un groupe $NR^{83}R^{84}$, un groupe alkyle en $C_1$ à $C_6$ (saturé), un groupe alkoxy en $C_1$ à $C_6$, un groupe cycloalkoxy en $C_3$ à $C_8$, un groupe cycloalkyle en $C_3$ à $C_8$ ou un groupe alkylène en $C_2$ à $C_6$,

le reste $R^{82}$ représentant H, un radical alkyle en $C_1$ à $C_{10}$, avantageusement alkyle en $C_1$ à $C_6$, un radical aryle ou hétéroaryle ou un reste aryle ou hétéroaryle lié par l'intermédiaire d'un radical alkyle en $C_1$ à $C_3$, saturé ou non saturé, ou par l'intermédiaire d'un radical alkylène en $C_1$ à $C_3$, ces restes aryle et hétéroaryle ne pouvant pas eux-mêmes être substitués avec des restes aryle ou hétéroaryle,

les restes $R^{83}$ et $R^{84}$ identiques ou différents, représentent H, un reste alkyle en $C_1$ à $C_{10}$, avantageusement un reste alkyle en $C_1$ à $C_6$, un reste aryle, un reste hétéroaryle ou un reste aryle ou hétéroaryle lié par l'intermédiaire d'un groupe alkyle en $C_1$ à $C_3$, saturé ou non saturé, ou d'un groupe alkylène en $C_1$ à $C_3$, ces restes aryle et hétéroaryle ne pouvant pas être substitués eux-mêmes avec des restes aryle ou hétéroaryle,

ou bien les restes $R^{83}$ et $R^{84}$ forment conjointement un groupe $CH_2CH_2OCH_2CH_2$, $CH_2CH_2NR^{85}CH_2CH_2$ ou $(CH_2)_{3-6}$, et

le reste $R^{85}$ représente H, un reste alkyle en $C_1$ à $C_{10}$, avantageusement un reste alkyle en $C_1$ à $C_6$, un reste aryle ou hétéroaryle ou un reste aryle ou hétéroaryle lié par l'intermédiaire d'un groupe alkyle en $C_1$ à $C_3$ saturé ou non saturé ou d'un groupe alkylène en $C_1$ à $C_3$, ces restes aryle et hétéroaryle ne pouvant pas eux-mêmes être substitués avec des restes aryle ou hétéroaryle, le cas échéant sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates.

2. Dérivés substitués de 4-aminocyclohexanol suivant la revendication 1, **caractérisés en ce que** $R^1$ et $R^2$ sont choisis indépendamment l'un de l'autre entre H ; un reste alkyle en $C_1$ à $C_8$, saturé ou non saturé, ramifié ou non ramifié, substitué une ou plusieurs fois ou non substitué ; $R^1$ et $R^2$ ne devant pas représenter tous deux H,

ou bien les restes $R^1$ et $R^2$ forment ensemble un noyau et représentent $CH_2CH_2OCH_2CH_2$, $CH_2CH_2NR^5CH_2CH_2$ ou $(CH_2)_{3-6}$,
$R^5$ étant choisi entre H ; un radical alkyle en $C_1$ à $C_8$, saturé ou non saturé, ramifié ou non ramifié, substitué une ou plusieurs fois ou non substitué,
avantageusement
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué ; $R^1$ et $R^2$ ne devant pas représenter tous deux H,
ou bien les restes $R^1$ et $R^2$ forment ensemble un noyau et représentent $(CH_2)_{4-5}$,
en particulier
$R^1$ et $R^2$ sont choisis indépendamment l'un de l'autre entre un reste méthyle ou éthyle ou bien les restes $R^1$ et $R^2$ forment ensemble un noyau et représentent $(CH_2)_5$.

3. Dérivés substitués de 4-aminocyclohexanol suivant l'une des revendications 1 ou 2, **caractérisés en ce que**
$R^3$ est choisi entre un reste cycloalkyle en $C_5$ ou $C_6$, non substitué ou substitué une ou plusieurs fois ; ou bien un reste, lié par l'intermédiaire d'un groupe alkyle en $C_1$ ou $C_2$ saturé, non ramifié, cycloalkyle en $C_5$ ou $C_6$, phényle, naphtyle, anthracényle, thiophényle, benzothiophényle, pyridyle, furyle, benzofurannyle, benzodioxolannyle, indolyle, indanyle, benzodioxannyle, pyrrolyle, pyrimidyle ou pyrazinyle, chacun non substitué ou substitué une ou plusieurs fois ;
en particulier
$R^3$ est choisi entre un reste, lié par l'intermédiaire d'un groupe alkyle en $C_1$ ou $C_2$ saturé non ramifié, phényle, pyridyle, furyle ou thiophényle, chacun non substitué ou substitué une ou plusieurs fois.

4. Dérivés substitués de 4-aminocyclohexanol suivant l'une des revendications 1 à 3, **caractérisés en ce que**
$R^4$ est choisi entre un reste cycloalkyle en $C_3$ à $C_8$, aryle ou hétéroaryle, chacun non substitué ou substitué une ou plusieurs fois ; ou un groupe $-R^8-L-R^9$,
avantageusement
$R^4$ est choisi entre un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle, ou pyrimidyle, fluorényle, fluoranthényle, benzothiazolyle, benzotriazolyle ou benzo[1,2,5]thiazolyle ou 1,2-dihydroacénaphténylе, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une
ou plusieurs fois ; ou un groupe $-R^8-L-R^9$,
en particulier
$R^4$ est choisi entre un reste cyclopentyle, cyclohexyle, cycloheptyle, cyclo-octyle, anthracényle, indolyle, naphtyle, benzothiazolyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois ; ou un groupe $-R^8-L-R^9$.

5. Dérivés substitués de 4-aminocyclohexanol suivant la revendication 4, **caractérisés en ce que**
$R^8$ est choisi entre
un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphténylе, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois,
L est choisi entre

$$-C(O)-NH-, -NH-C(O)-, -C(O)-O-, -O-C(O)-, -O-, -S- \text{ ou } -S(O)_2-,$$

et/ou $R^9$ est choisi entre
un reste indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphténylе, pyridinyle, furannyle, benzofurannyle, pyrazolinonyle, oxopyrazolinonyle, pyrimidinyle, quinolinyle, isoquinolinyle, phtalazinyle ou quinazolinyle, chacun non substitué ou substitué une ou plusieurs fois,
avantageusement

$R^8$ est choisi entre
un reste indolyle, benzothiophényle, phényle, thiophényle, furyle, pyridyle, pyrrolyle, pyrazinyle ou pyrimidyle, chacun non substitué ou substitué une ou plusieurs fois,
L est choisi entre

-C(O)-NH-, -NH-C(O)-, -C(O)-O-, -O-C(O)- ou $-S(O)_2-$,

et/ou $R^9$ est choisi entre
un reste indolyle, benzothiophényle, phényle, thiophényle, furyle, pyridyle, pyrrolyle, pyrazinyle ou pyrimidyle, chacun non substitué ou substitué une ou plusieurs fois,
en particulier
$R^8$ est choisi comme
reste indolyle non substitué,
L est choisi comme
groupe $-S(O)_2-$
et $R^9$ est choisi comme
reste phényle non substitué.

6. Dérivés substitués de 4-aminocyclohexanol suivant l'une des revendications 1 à 3, **caractérisés en ce que**
$R^4$ est choisi entre un groupe $-CHR^6R^7$, $-CHR^6-CH_2R^7$, $-CHR^6-CH_2-CH_2R^7$, $-CHR^6-CH_2-CH_2-CH_2R^7$, $-C(Y)R^7$, -C (Y)-$CH_2R^7$, $-C(Y)-CH_2-CH_2R^7$ ou $-C(Y)-CH_2-CH_2-CH_2R^7$
avec Y = O, S ou $H_2$,
avantageusement
$R^4$ est choisi entre un groupe $-CHR^6R^7$, $-CHR^6CH_2R^7$, $-CHR^6-CH_2-CH_2R^7$, $-C(Y)R^7$, $-C(Y)-CH_2R^7$ ou -C (Y) $-CH_2-CH_2R^7$ avec Y = O ou S,
en particulier
$R^4$ est choisi entre un groupe $-CHR^6R^7$, $-CHR^6-CH_2R^7$, $-C(Y)R^7$ ou $-C(Y)-CH_2R^7$
avec Y = O.

7. Dérivés substitués de 4-aminocyclohexanol suivant la revendication 6, **caractérisés en ce que**
$R^6$ est choisi entre
H, un reste alkyle en $C_1$ à $C_4$, saturé ou non saturé, ramifié ou non ramifié, substitué une ou plusieurs fois ou non substitué ; ou un groupe C-(O)O-(alkyle en $C_1$ à $C_4$), saturé ou non saturé, ramifié ou non ramifié, substitué une ou plusieurs fois ou non substitué ;
avantageusement
H, un reste alkyle en $C_1$ à $C_4$, saturé ou non saturé, ramifié ou non ramifié, substitué une ou plusieurs fois ou non substitué ;
en particulier
H, $CH_3$ et $C_2H_5$.

8. Dérivés substitués de 4-aminocyclohexanol suivant la revendication 6, **caractérisés en ce que**
$R^7$ est choisi entre un reste cycloalkyle en $C_3$ à $C_8$, aryle ou hétéroaryle, chacun non substitué ou substitué une ou plusieurs fois ; avantageusement
$R^7$ est choisi entre un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou substitué une ou plusieurs fois ; en particulier
$R^7$ est choisi entre un reste cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

9. Dérivés substitués de 4-aminocyclohexanol suivant l'une des revendications 1 à 8, **caractérisés en ce qu'**ils sont choisis dans le groupe suivant :

- 4-benzyl-4-diméthylamino-1-phénéthylcyclohexanol ainsi que le chlorhydrate correspondant,

- 4-diméthylamino-1,4-diphénéthylcyclohexanol ainsi que le chlorhydrate correspondant,
- 4-benzyl-4-diméthylamino-1-[2-(2-fluorophényl)-éthyl]-cyclohexanol ainsi que le chlorhydrate correspondant,
- 4-benzyl-9-diméthylamino-1-[2-(4-fluorophényl)-éthyl]-cyclohexanol ainsi que le chlorhydrate correspondant,
- 4-diméthylamino-4-(2-fluorobenzyl)-1-phénéthylcyclohexanol ainsi que le chlorhydrate correspondant,
- 4-diméthylamino-4-(3-fluorobenzyl)-1-phénéthylcyclohexanol ainsi que le chlorhydrate correspondant,
- 4-diméthylamino-4-(4-fluorobenzyl)-1-phénéthylcyclohexanol ainsi que le chlorhydrate correspondant,
- 4-benzyl-4-diméthylamino-1-[2-(3-fluorophényl)-éthyl]-cyclohexanol ainsi que le chlorhydrate correspondant,
- 4-benzyl-4-diméthylamino-1-(2-fluorobenzyl)-cyclohexanol ainsi que le chlorhydrate correspondant,
- 4-(allylméthylamino)-4-benzyl-1-phénéthylcyclohexanol ainsi que le chlorhydrate correspondant,
- 4-benzyl-4-diméthylamino-1-(3-fluorobenzyl)-cyclohexanol ainsi que le chlorhydrate correspondant,
- 4-benzyl-4-diméthylamino-1-(4-fluorobenzyl)-cyclohexanol ainsi que le chlorhydrate correspondant,
- 1-benzyl-4-diméthylamino-4-(3-fluorobenzyl)-cyclohexanol ainsi que le chlorhydrate correspondant ou
- 4-benzyl-1-phénéthyl-4-pyrrolidine-1-ylcyclohexanol ainsi que le chlorhydrate correspondant,
- 4-benzyl-4-diméthylamino-1-(1-méthyl-1H-indole-2-yl)-cyclohexanol
- 1-benzo[b]thiophène-2-yl-4-benzyl-4-diméthylaminocyclohexanol
- 1-benzo[b]thiophène-3-yl-4-benzyl-4-diméthylaminocyclohexanol
- 1-benzofuranne-2-yl-4-benzyl-4-diméthylaminocyclohexanol éventuellement sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque, sous la forme représentée ou sous forme de leurs acides ou de leurs bases ou sous forme de leurs sels, en particulier des sels physiologiquement acceptables, ou sous forme de leurs produits de solvatation, en particulier des hydrates.

**10.** Médicament contenant au moins un dérivé substitué de 4-aminocyclohexanol suivant l'une des revendications 1 à 9, éventuellement sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme des acides ou des bases ou sous forme des sels, en particulier des sels physiologiquement acceptables, ou sous forme des produits de solvatation, en particulier des hydrates ; ainsi que, le cas échéant, des additifs et/ou des substances auxiliaires convenables et/ou le cas échéant d'autres substances actives.

**11.** Médicament suivant la revendication 10, **caractérisé en ce qu'**il contient, en plus d'au moins un dérivé substitué de 4-aminocyclohexanol, un opiacé, avantageusement un opiacé puissant, en particulier la morphine, ou un anesthésique, avantageusement de l'hexobarbital ou de l'halothane.

**12.** Utilisation d'un dérivé substitué de 4-aminocyclohexanol suivant l'une des revendications 1 à 9, éventuellement sous forme de ses racémates, de ses stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de ses acides ou de ses bases ou sous forme de ses sels, en particulier des sels physiologiquement acceptables, ou sous forme de ses produits de solvatation, en particulier des hydrates ; pour la préparation d'un médicament destiné au traitement de la douleur, en particulier d'une douleur aiguë, viscérale, neuropathique ou chronique.

**13.** Utilisation d'un dérivé substitué de 4-aminocyclohexanol suivant l'une des revendications 1 à 9, éventuellement sous forme de ses racémates, de ses stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de ses acides ou de ses bases ou sous forme de ses sels, en particulier des sels physiologiquement acceptables, ou sous forme de ses produits de solvatation, en particulier des hydrates ; pour la préparation d'un médicament destiné au traitement d'états d'anxiété, de stress et de syndromes liés au stress, de dépressions, de l'épilepsie, de la maladie d'Alzheimer, de la démence sénile, de troubles cognitifs généraux, de difficultés d'apprentissage et de mémoire (comme nootrope), de phénomènes de privation, d'abus d'alcools et/ou de drogues et/ou de médicaments et/ou de pharmacodépendance, de dysfonctionnements sexuels, de maladies cardio-vasculaires, d'hypotension, d'hypertension, d'acouphènes, de prurit, de migraine, de surdité, d'insuffisance de la motilité intestinale, de troubles de l'ingestion de nourriture, d'anorexie, d'obésité, de troubles locomoteurs, de diarrhée, de cachexie, d'incontinence d'urine ou comme myorelaxant, anticonvulsif, antitussif ou anesthésique ou en vue d'une coadministration en cas de traitement avec un analgésique opiacé ou avec un anesthésique, pour la diurèse ou l'antinatriurèse et/ou pour l'anxiolyse.

**14.** Procédé de production d'un dérivé substitué de 4-aminocyclohexanol suivant l'une des revendications 1 à 9, comprenant les étapes suivantes :

a. une cyclohexane-1,4-dione protégée avec les groupes $S^1$ et $S^2$ selon la formule II est amenée à réagir en présence d'un composé de formule $HNR^{01}R^{02}$ avec un cyanure, avantageusement le cyanure de potassium, pour former un dérivé de 1-amino-4-oxocyclohexanecarbonitrile protégé N-substitué répondant à la formule III ;

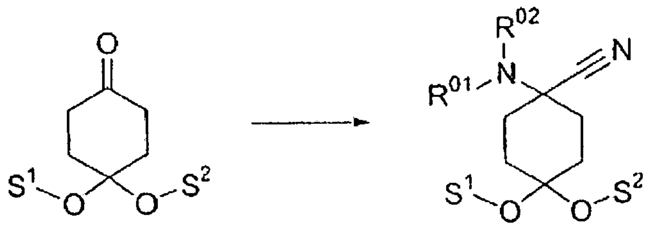

II III

on effectue ensuite éventuellement, dans un ordre quelconque et le cas échéant de façon répétée, une acylation, une alkylation ou une sulfonation et/ou dans le cas de composés dans lesquels $R^{01}$ et/ou $R^{02}$ et/ou $R^{06}$ représentent H protégé par un groupe protecteur, on élimine au moins une fois un groupe protecteur et on effectue éventuellement une acylation, une alkylation ou une sulfonylation et/ou dans le cas d'un composé dans lequel $R^{01}$ et/ou $R^{02}$ et/ou $R^{06}$ représentent H, on introduit au moins une fois un groupe protecteur et on effectue éventuellement une acylation, une alkylation ou une sulfonylation,

b. l'aminonitrile de formule III est amené à réagir avec des réactifs organométalliques, avantageusement des réactifs de Grignard ou des composés organiques de lithium, de formule métal-$R^3$, de manière à produire un composé de formule IVa ;

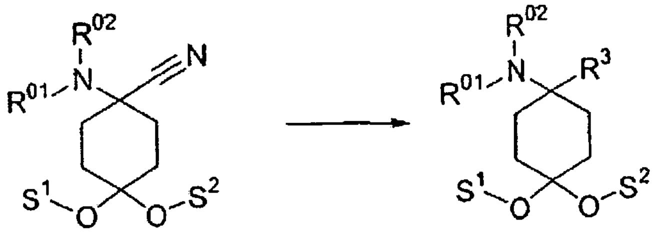

III IVa

on effectue ensuite éventuellement, dans un ordre quelconque et le cas échéant de façon répétée, une acylation, une alkylation ou une sulfonylation et/ou dans le cas de composés dans lesquels $R^{01}$ et/ou $R^{02}$ et/ou $R^{06}$ représentent H protégé par un groupe protecteur, on élimine au moins une fois un groupe protecteur et, le cas échéant, on effectue une acylation, une alkylation ou une sulfonylation et/ou dans le cas de composés dans lesquels $R^{01}$ et/ou $R^{02}$ et/ou $R^{06}$ représentent H, on introduit au moins une fois un groupe protecteur et on effectue éventuellement une acylation, une alkylation ou une sulfonylation,

c. sur le composé de formule IVa selon la formule III, les groupes protecteurs $S^1$ et $S^2$ sont éliminés, ce qui donne un dérivé 4-substitué de 4-aminocyclohexanone répondant à la formule IV ;

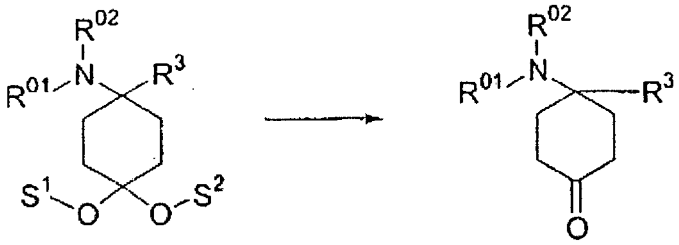

IVa IV

on conduit ensuite éventuellement, dans un ordre quelconque et, le cas échéant, de façon répétée, une acylation, une alkylation ou une sulfonylation et/ou dans le cas de composés dans lesquels $R^{01}$ et/ou $R^{02}$ et/ou $R^{06}$ représentent H protégé par un groupe protecteur, on élimine au moins une fois un groupe protecteur et on effectue le cas échéant une acylation, une alkylation ou une sulfonylation et/ou dans le cas de composés dans lesquels $R^{01}$ et/ou $R^{02}$ et/ou $R^{06}$ représentent H, on introduit au moins une fois un groupe protecteur et on effectue éventuellement une acylation, une alkylation ou une sulfonylation,

d. le dérivé 4-substitué de 4-aminocyclohexanone selon la formule IV est amené à réagir avec des réactifs organométalliques, avantageusement les réactifs de Grignard ou des composés organiques de lithium, de formule métal-$R^3$, de manière à produire un composé de formule V ;

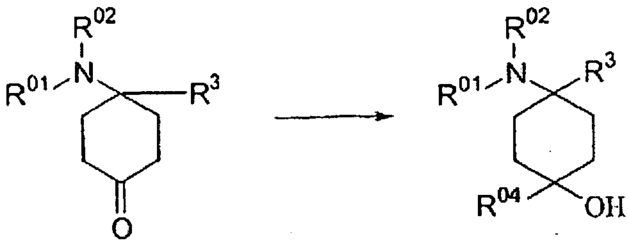

IV V

on conduit ensuite éventuellement dans un ordre quelconque et, le cas échéant, de façon répétée, une acylation, une alkylation ou une sulfonylation et/ou dans le cas de composés dans lesquels $R^{01}$ et/ou $R^{02}$ et/ou $R^{04}$ et/ou $R^{05}$ et/ou $R^{06}$ représentent H protégé par un groupe protecteur, on élimine au moins une fois un groupe protecteur et on effectue éventuellement une acylation, une alkylation ou une sulfonylation et/ou dans le cas de composés dans lesquels $R^{01}$ et/ou $R^{02}$ et/ou $R^{04}$ et/ou $R^{05}$ et/ou $R^{06}$ représentent H, on introduit au moins une fois un groupe protecteur et on effectue le cas échéant une acylation, une alkylation

ou une sulfonylation, jusqu'à ce qu'on obtienne un composé de formule I,

dans laquelle $R^1$, $R^2$, $R^3$ $R^4$ et $R^5$ ont la définition indiquée dans la revendication 1 et

$R^{01}$ et $R^{02}$ sont choisis indépendamment l'un de l'autre entre H ; H pourvu d'un groupe protecteur ; un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

ou bien les restes $R^{01}$ et $R^{02}$ forment ensemble un noyau et représentent $CH_2CH_2OCH_2CH_2$, $CH_2CH_2NR^{05}CH_2CH_2$ ou $(CH_2)_{3-6}$,

$R^{05}$ étant choisi entre H ; H pourvu d'un groupe protecteur ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un groupe alkylène en $C_1$ à $C_3$, aryle, cycloalkyle en $C_3$ à $C_8$ ou hétéroaryle, chacun substitué une ou plusieurs fois ou non substitué ;

$R^{04}$ est choisi entre H, H pourvu d'un groupe protecteur ; un reste cycloalkyle en $C_3$ à $C_8$, aryle ou hétéroaryle, chacun non substitué ou substitué une ou plusieurs fois ; un groupe $-CHR^6R^7$, $-CHR^6-CH_2R^7$, $-CHR^6-CH_2-CH_2R^7$,

$-CHR^6-CH_2CH_2-CH_2R^7$, $-C(Y)R^7$, $-C (Y)-CH_2R^7$, $-C(Y)-CH_2-CH_2R^7$ ou $-C(Y)-CH_2-CH_2-CH_2R^7$ ; ou $-R^6-L-R^9$
avec Y = O, S ou $H_2$,
$R^6$ est choisi entre H, un reste alkyle en $C_1$ à $C_7$, saturé ou non saturé, ramifié ou non ramifié, substitué une ou plusieurs fois ou non substitué ; ou un groupe C(O)O-, un reste alkyle en $C_1$ à $C_6$, saturé ou non saturé, ramifié ou non ramifié, substitué une ou plusieurs fois ou non substitué ;
et $R^7$ étant choisi entre
H ; un reste cycloalkyle en $C_3$ à $C_8$, aryle ou hétéroaryle, chacun non substitué ou substitué une ou plusieurs fois,
$R^8$ étant choisi entre
un reste aryle ou hétéroaryle, chacun non substitué ou substitué une ou plusieurs fois,
L étant choisi entre
-C(O)-NH-, -NH-C(O)-, -C(O)-O-, -O-C(O)-, -O-, -S- ou $-S(O)_2-$
$R^9$ étant choisi entre
un reste aryle ou hétéroaryle, chacun non substitué ou substitué une ou plusieurs fois,
et $S^1$ et $S^2$ sont choisis indépendamment l'un de l'autre entre des groupes protecteurs ou forment ensemble un groupe protecteur, avantageusement un groupe monoacétal.

**15.** Procédé de production d'un dérivé substitué de 4-aminocyclohexanol suivant la revendication 14, **caractérisé en ce que** les groupes protecteurs concernant H dans le cas de $R^{01}$, $R^{02}$, $R^{04}$ et/ou $R^{05}$ sont choisis entre des groupes alkyle, benzyle ou carbamate, par exemple FMOC, Z ou Boc.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2839891 A
- US 4366172 A


### In der Beschreibung aufgeführte Nicht-Patentliteratur


- **MEUNIER et al.** *Nature,* 1995, vol. 377, 532-535
- **MOLLEREAU et al.** *FEBS Letters,* 1994, vol. 341, 33-38
- **DARLAND et al.** *Trends in Neurosciences,* 1998, vol. 21, 215-221
- **ARDATI et al.** *Mol. Pharmacol.,* vol. 51, 816-824
- **MATTHES et al.** *Mol. Pharmacol.,* 1996, vol. 50, 447-450
- **VAUGHAN et al.** *Br. J. Pharmacol.,* 1996, vol. 117, 1609-1611
- **CONNER et al.** *Br. J. Pharmacol.,* 1996, vol. 118, 205-207
- **KNOFLACH et al.** *J. Neuroscience,* 1996, vol. 16, 6657-6664
- **REINSCHEID et al.** *Science,* 1995, vol. 270, 792-794
- **HARA et al.** *Br. J. Pharmacol.,* 1997, vol. 121, 401-408
- **MOGIL et al.** *Neurosci. Letters,* 1996, vol. 214, 131-134
- *Neuroscience,* 1996, vol. 75, 333-337
- **JENCK et al.** *Proc. Natl. Acad. Sci. USA,* 1997, vol. 94, 14854-14858
- **SHU et al.** *Neuropeptides,* 1998, vol. 32, 567-571
- **FABER et al.** *Br. J. Pharmacol.,* 1996, vol. 119, 189-190
- **KING et al.** *Neurosci. Lett.,* 1997, vol. 223, 113-116
- **XU et al.** *NeuroReport,* 1996, vol. 7, 2092-2094
- **YAMAMOTO et al.** *Neuroscience,* 1997, vol. 81, 249-254
- **YAMAMOTO ; NOZAKI-TAGUCHI.** *Anesthesiology,* 1997, vol. 87
- **ABDULLA ; SMITH.** *J. Neurosci.,* 1998, vol. 18, 9685-9694
- **SANDIN et al.** *Eur. J. Neurosci.,* 1997, vol. 9, 194-197
- **MANABE et al.** *Nature,* 1997, vol. 394, 577-581
- **NISHI et al.** *EMBO J.,* 1997, vol. 16, 1858-1864
- **POMONIS et al.** *NeuroReport,* 1996, vol. 8, 369-371
- **GUMUSEL et al.** *Life Sci.,* 1997, vol. 60, 141-145
- **CAMPION ; KADOWITZ.** *Biochem. Biophys. Res. Comm.,* 1997, vol. 234, 309-312
- **GUTIÉRREZ et al.** *Society for Neuroscience,* 07. November 1998, vol. 24
- **KAPISTA et al.** *Life Sciences,* 1997, vol. 60, 15-21
- **CALO et al.** *Br.J. Pharmacol.,* 2000, vol. 129, 1261-1283
- **D. LEDNICER ; P.F. VON VOIGHTLANDER.** *J. Med. Chem.,* 1979, vol. 22, 1157
- **D. LEDNICER ; P.F. VON VOIGHTLANDER ; D.E. EMMERT.** *J. Med. Chem.,* 1980, vol. 23, 424
- **D. LEDNICER ; P.F. VON VOIGHTLANDER ; D.E. EMMERT.** *J. Med. Chem.,* 1981, vol. 24, 404
- **D. LEDNICER ; P.F. VON VOIGHTLANDER ; D.E. EMMERT.** *J. Med. Chem.,* 1981, vol. 24, 340
- Endogenous and Exogenous Opiate Agonists and Antagonists. **P.F. VONVOIGHTLANDER ; D. LEDNICER ; R.A. LEWIS ; D.D. GAY.** Proc. Int. Narc. Res. Club Conf. (1980), Meeting Date 1979. Pergamon, Elmsford, N.Y.International, Pergamon, 1980, 17-21
- **KAMENKA et al.** *EurJ.Med.Chem.Chim.Ther.,* 1984, vol. 19 (3), 255-260
- **RAO M.N.A. ; RAO S.C.** *Indian Drugs,* 1985, vol. 22 (5), 252-257
- **ARDATI et al.** *Mol. Pharmacol.,* 1997, vol. 51, 816-824
- **D'AMOUR ; SMITH.** *J. Pharm. Exp. Ther.,* 1941, vol. 72, 74 79